# EUROPÄISCHE PATENTSCHRIFT

(11) **EP 0 772 604 B1**
(45) Veröffentlichungstag und Bekanntmachung des Hinweises auf die Patenterteilung: **06.05.1999**
(21) Anmeldenummer: 95926895.4
(22) Anmeldetag: 14.07.1995
(51) Int. Cl.: C07D 249/12, C07D 249/14, C07D 249/08, C07D 401/06, C07D 401/12, C07D 403/12, C07D 401/14, A61K 31/41, A61K 31/505, A61K 31/435, A61K 31/53

(54) **TRIAZOLVERBINDUNGEN UND DEREN VERWENDUNG ALS DOPAMIN-D3-LIGANDEN**
TRIAZOLE COMPOUNDS AND THEIR USE AS DOPAMINE-D3-LIGANDS
COMPOSES TRIAZOLE ET LEUR UTILISATION COMME LIGANDS DE LA DOPAMINE-D3

(30) Priorität: 15.07.1994 DE 4425144
(43) Veröffentlichungstag der Anmeldung: 14.05.1997
(73) Patentinhaber: BASF AKTIENGESELLSCHAFT, 67056 Ludwigshafen (DE)
(72) Erfinder: HELLENDAHL, Beate, D-67105 Schifferstadt (DE); LANSKY, Annegret, D-64297 Darmstadt (DE); MUNSCHAUER, Rainer, Shrewsbury, MA 01545 (US); BIALOJAN, Siegfried, D-68723 Oftersheim (DE); UNGER, Liliane, D-67065 Ludwigshafen (DE); TESCHENDORF, Hans-Jürgen, D-67373 Dudenhofen (DE); WICKE, Karsten, D-67122 Altrip (DE); DRESCHER, Karla, D-69221 Dossenheim (DE)
(74) Vertreter: Kinzebach, Werner, Dr.
(86) Internationale Anmeldenummer: EP9502781
(87) Internationale Veröffentlichungsnummer: WO9602520

(56) Entgegenhaltungen:
- FR-A- 2 551 439
- FR-A- 2 552 759
- US-A- 4 577 020
- JOURNAL OF MEDICINAL CHEMISTRY, Bd. 37, Nr. 8, 15.April 1994 WASHINGTON DC US, Seiten 1060-2, A.B. REITZ ET AL. 'A new arylpiperazine antipsychotic with high D2/D3/5-HT1A/alpha1A-adrenergic affinity and a low potential for extrapyramidal effects'

## Beschreibung

Die Erfindung betrifft Triazolverbindungen und die Verwendung derartiger Verbindungen. Die erwähnten Verbindungen besitzen wertvolle therapeutische Eigenschaften und sind zur Behandlung von Erkrankungen brauchbar, die auf Dopamin-D₃-Rezeptorliganden ansprechen.

Verbindungen der hier in Rede stehenden Art mit physiologischer Aktivität sind bereits bekannt. Die US-A- 4,338,453; 4,408,049 und 4,577,020 beschreiben Triazolverbindungen, welche anti-allergische Aktivität besitzen.

Die FR-A-2 551 439 beschreibt Verbindungen der Formel: 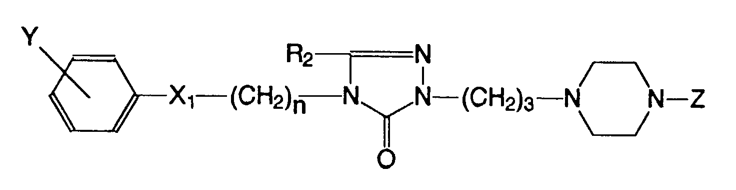 worin
- n: für 2 bis 4 steht,
- R₂: für ein Wasserstoffatom oder eine Niedrigalkylgruppe steht,
- X₁: für Sauerstoff oder eine Bindung steht, Y für Wasserstoff, Halogen, Niedrigalkoxy oder CF₃ steht, und
- Z: für einen durch Halogen oder CF₃ substituierten Phenylrest oder für einen gegebenenfalls substituierten 2-Pyridin-, 2-Pyrimidin-, 3-Pyrimidin-, 3-Pyridazin-, 2-Chinolin- oder 3-Benzothiazolrest steht.

Diese Verbindungen sind antidepressiv wirksam.

In J.Med.Chem. 1994, 37, 1060-1062, wird die Verbindung der Formel: 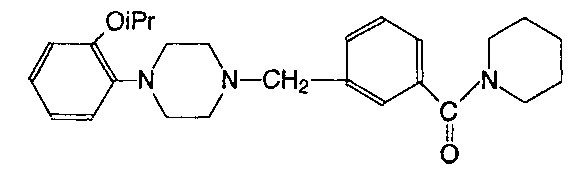 beschrieben. Sie bindet mit hoher Affinität an D₂, D₃, 5-HT_{1A} und α_{1A}-adrenerge Rezeptoren und zeigt hohe und selektive Aktivität in Tiermodellen, die auf antipsychotische Aktivität beim Menschen schließen lassen.

Neuronen erhalten ihre Informationen unter anderem über G-Protein-gekoppelte Rezeptoren. Es gibt zahlreiche Substanzen, welche ihre Wirkung über diese Rezeptoren ausüben. Eine davon ist Dopamin.

Es liegen gesicherte Erkenntnisse über die Anwesenheit von Dopamin und dessen physiologische Funktion als Neurotransmitter vor. Auf Dopamin ansprechende Zellen stehen im Zusammenhang mit der Etiologie von Schizophrenie und der Parkinson'schen Krankheit. Die Behandlung dieser und anderer Erkrankungen erfolgt mit Arzneimitteln, die mit den Dopaminrezeptoren in Wechselwirkung treten.

Bis 1990 waren zwei Subtypen von Dopaminrezeptoren pharmakologisch klar definiert, nämlich die D₁- und D₂-Rezeptoren.

Sokoloff et al., Nature 1990, 347 : 146-151, haben einen dritten Subtyp gefunden, nämlich die D₃-Rezeptoren. Sie werden hauptsächlich im limbischen System exprimiert. Strukturell unterscheiden sich die D₃-Rezeptoren von den D₁- und D₂-Rezeptoren in etwa der Hälfte der Aminosäurereste.

Die Wirkung von Neuroleptika wurde im allgemeinen ihrer Affinität zu den D₂-Rezeptoren zugeschrieben. Neuere Rezeptorverbindungsstudien haben dies bestätigt. Danach besitzen die meisten Dopaminantagonisten, wie Neuroleptika, hohe Affinität zu den D₂-Rezeptoren, aber nur geringe Affinität zu den D₃-Rezeptoren.

Überraschenderweise wurde nun gefunden, daß die erfindungsgemäßen Verbindungen eine hohe Affinität zum Dopamin-D₃-Rezeptor und eine nur geringe Affinität zum D₂-Rezeptor aufweisen. Es handelt sich somit um selektive D₃-Liganden.

Gegenstand der vorliegenden Erfindung sind daher Triazolverbindungen der Formel I: 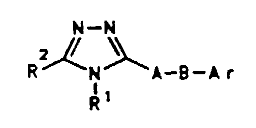 worin
- A: für eine geradkettige oder verzweigte C₁-C₁₈-Alkylengruppe steht, die gegebenenfalls wenigstens eine Gruppe umfassen kann, die ausgewählt ist unter O, S, NR³, CONR³, NR³CO, COO, OCO, C₃-C₆-Cycloalkylen oder einer Doppel- oder Dreifachbindung,
- B: für einen Rest der Formel steht: 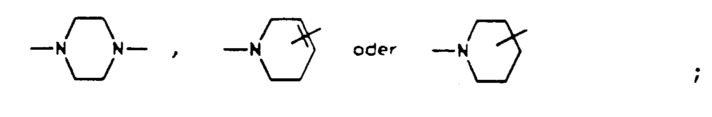
R¹ für H, CO₂R³, NR³R⁴, OR⁴, C₃-C₆-Cycloalkyl oder C₁-C₈-Alkyl, das gegebenenfalls durch OH, OC₁-C₈-Alkyl oder Halogen substituiert ist, steht;
- R²: die für R¹ angegebenen Bedeutungen besitzt oder für CF₃, SR³, Halogen oder CN steht;
- R³: für H oder C₁-C₈-Alkyl, das gegebenenfalls durch OH, OC₁-C₈-Alkyl, Phenyl oder Halogen substituiert ist, steht;
- R⁴: die für R³ angegebenen Bedeutungen besitzt oder für COR³ oder CO₂R³ steht;
- Ar: für Phenyl, Pyridyl, Pyrimidyl oder Triazinyl steht, wobei Ar gegebenenfalls ein bis vier Substituenten aufweisen kann, die unabhängig voneinander ausgewählt sind unter OR⁴, C₁-C₈-Alkyl, C₂-C₆-Alkenyl, C₂-C₆-Alkinyl, Halogen, CN, CO₂R³, NO₂, SO₂R³, SO₃R³, NR³R⁴, SO₂NR³R⁴, SR³, CF₃, CHF₂, einem 5- oder 6-gliedrigen carbocyclischen, aromatischen oder nicht-aromatischen Ring und einem 5- oder 6-gliedrigen heterocyclischen aromatischen oder nicht-aromatischen Ring mit 1 bis 4 Heteroatomen, die ausgewählt sind unter 0, S und N, wobei der carbocyclische oder heterocyclische Ring gegebenenfalls substituiert sein kann durch C₁-C₈-Alkyl, Halogen, OC₁-C₈-Alkyl, OH, NO₂ oder CF₃ und wobei Ar gegebenenfalls auch mit einem carbocyclischen oder heterocyclischen Ring der oben definierten Art kondensiert sein kann, ausgenommen die Verbindung der Formel: 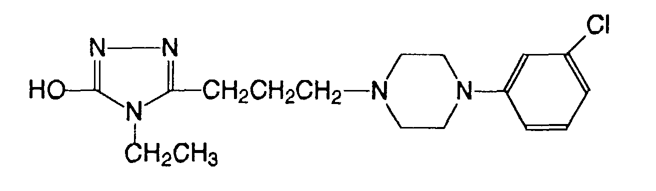
sowie deren Salze mit physiologisch verträglichen Säuren.

Bei den erfindungsgemäßen Verbindungen handelt es sich um selektive Dopamin-D₃-Rezeptor-Liganden, die regioselektiv im limbischen System angreifen und aufgrund ihrer geringen Affinität zum D₂-Rezeptor nebenwirkungsärmer als klassische Neuroleptika sind bei denen es sich um D₂-Rezeptorantagonisten handelt. Die Verbindungen sind daher zur Behandlung von Erkrankungen brauchbar, die auf Dopamin-D₃-Rezeptorantagonisten bzw. -agonisten ansprechen, z.B. zur Behandlung von Erkrankungen des zentralen Nervensystems, insbesondere Schizophrenie, Depressionen, Neurosen, und Psychosen. Außerdem sind sie zur Behandlung von Schlafstörungen und Übelkeit und als Antihistaminika brauchbar.

Im Rahmen der vorliegenden Erfindung besitzen die nachfolgenden Ausdrücke die anschließend angegebenen Bedeutungen:

Alkyl (auch in Resten wie Alkoxy, Alkylamino etc) bedeutet eine geradkettige oder verzweigte Alkylgruppe mit 1 bis 8 Kohlenstoffatomen, vorzugsweise 1 bis 6 Kohlenstoffatomen und insbesondere 1 bis 4 Kohlenstoffatomen. Die Alkylgruppe kann einen oder mehrere Substituenten aufweisen, die unabhängig voneinander ausgewählt sind unter OH und OC₁-C₈-Alkyl.

Beispiele für eine Alkylgruppe sind Methyl, Ethyl, n-Propyl, i-Propyl, n-Butyl, iso-Butyl, t-Butyl etc.

Alkylen steht für geradkettige oder verzweigte Reste mit vorzugsweise 2 bis 15 Kohlenstoffatomen, besonders bevorzugt 3 bis 10 Kohlenstoffatomen.

Die Alkylengruppen können gegebenenfalls wenigstens eine der oben angegebenen Gruppen umfassen. Diese kann - ebenso wie die erwähnte Doppel- oder Dreifachbindung - in der Alkylenkette an beliebiger Stelle oder an dem Ende der Kette angeordnet sein so, daß sie die Kette mit dem Triazolrest verbindet. Letzteres ist bevorzugt. Wenn die Alkylengruppe eine Doppel- oder Dreifachbindung umfaßt, besitzt sie mindestens drei Kohlenstoffatome in der Kette.

Halogen bedeutet F, Cl, Br, I und insbesondere Cl, Br, I.

Vorzugsweise stehen R¹ und R² unabhängig voneinander für H, C₁-C₈-Alkyl, NR³R⁴ oder OR⁴.

Der Rest Ar kann einen, zwei, drei oder vier Substituenten aufweisen. Vorzugsweise sind sie unabhängig voneinander ausgewählt unter Halogen, CF₃, CHF₂, NR³R⁴, OR⁴, NO₂, C₁-C₈-Alkyl, OC₁-C₈-Alkyl, SR³ und CN, wobei R³ und R⁴ die oben angegebenen Bedeutungen besitzen.

Wenn einer der Substituenten des Restes Ar für C₁-C₈-Alkyl steht, ist ein verzweigter Rest, insbesondere die Isopropyl- oder t-Butyl-Gruppe bevorzugt.

Ar weist vorzugsweise mindestens einen Substituenten auf und steht insbesondere für 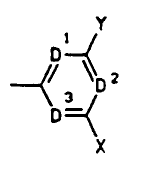 worin D¹, D² und D³ unabhängig voneinander für CR oder N stehen und R, X und Y für H oder die oben bzw. nachfolgend angegebenen Substituenten des Restes Ar stehen.

Vorzugsweise steht Ar für gegebenenfalls substituiertes Phenyl, 2-, 3- oder 4-Pyridinyl oder 2-, 4(6)- oder 5-Pyrimidinyl.

Wenn einer der Substituenten des Restes Ar für einen 5- oder 6-gliedrigen heterocyclischen Ring steht, so handelt es sich beispielsweise um einen Pyrrolidin-, Piperidin-, Morpholin-, Piperazin-, Pyridin-, Pyrimidin-, Triazin-, Pyrrol-, Thiophen-, Thiazol-, Imidazol-, Oxazol-, Isoxazol-, Pyrazol-, oder Thiadiazolrest.

Wenn einer der Substituenten des Restes Ar für einen carbocyclischen Rest steht, handelt es sich insbesondere um einen Phenyl-, Cyclopentyl- oder Cyclohexylrest.

Wenn Ar mit einem carbocyclischen oder heterocyclischen Rest kondensiert ist, steht Ar insbesondere für einen Naphthalin-, Di- oder Tetrahydronaphthalin-, Chinolin-, Di- oder Tetrahydrochinolin, Indol-, Dihydroindol-, Benzimidazol-, Benzothiazol-, Benzothiadiazol-, Benzopyrrol- oder Benzotriazolrest.

B steht vorzugsweise für

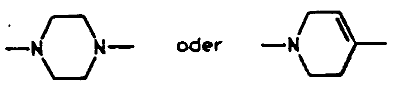

Eine bevorzugte Ausführungsform sind die Verbindungen der Formel I, worin A für C₃-C₁₀-Alkylen steht, das mindestens eine Gruppe umfaßt, die ausgewählt ist unter O, S, NR³, Cyclohexylen, insbesondere 1,4-Cyclohexylen, und einer Doppel- oder Dreifachbindung, wobei R³ wie oben definiert ist.

Eine weitere bevorzugte Ausführungsform sind die Verbindungen der Formel I, worin
- R¹: für H, OR⁴, wobei R⁴ für H oder C₁-C₈-Alkyl steht, C₃-C₆-Cycloalkyl oder C₁-C₈-Alkyl, das gegebenenfalls durch OH, OC₁-C₈-Alkyl oder Halogen substituiert ist, steht;
- R²: für H, C₁-C₈-Alkyl, das gegebenenfalls durch OH, OC₁-C₈-Alkyl oder Halogen substituiert ist, NR³R⁴, wobei R³ und R⁴ unabhängig voneinander für H, Phenyl-C₁-C₈-alkyl oder C₁-C₈-Alkyl stehen, OR⁴, wobei R⁴ für H oder C₁-C₈-Alkyl steht, oder CF₃ steht;
- A: wie in Anspruch 3 definiert ist, und
- Ar: für Phenyl, Pyridyl oder Pyrimidyl steht, das gegebenenfalls einen, zwei, drei oder vier Substituenten aufweist, die ausgewählt sind unter
H, C₁-C₈-Alkyl, das gegebenenfalls durch OH, OC₁-C₈-Alkyl oder Halogen substituiert ist, OR⁴, wobei R⁴ für H, C₁-C₈-Alkyl, das gegebenenfalls durch OH, OC₁-C₈-Alkyl oder Halogen substituiert ist, CHF₂, CF₃, CN, Halogen, C₂-C₆-Alkenyl, C₂-C₆-Alkinyl, Phenyl, Naphthyl und einem 5- oder 6-gliedrigen heterocyclischen aromatischen Rest mit 1 bis 3 Heteroatomen, ausgewählt unter O, N und S.

Eine weitere bevorzugte Ausführungsform sind die Verbindungen der Formel I, worin
- R¹: für H oder C₁-C₈-Alkyl, das gegebenenfalls substituiert ist durch OH, OC₁-C₈-Alkyl oder Halogen, steht;
- R²: für H, C₁-C₈-Alkyl, das gegebenenfalls substituiert ist durch OH, OC₁-C₈-Alkyl oder Halogen, NR³R⁴, wobei R³ und R⁴ unabhängig voneinander für H oder C₁-C₈-Alkyl stehen, OR⁴, wobei R⁴ für H oder C₁-C₈-Alkyl steht, oder CF₃ steht;
- A: für C₁-C₁₀-Alkylen steht, das gegebenenfalls ein Sauerstoff- oder Schwefelatom oder die Gruppe NR³ umfaßt, wobei R³ wie oben definiert ist;
- Ar: für Phenyl steht, das ein bis vier Substituenten aufweist, die unabhängig voneinander ausgewählt sind unter H, CN, SR³, Halogen, C₁-C₈-Alkyl, das gegebenenfalls durch OH, OC₁-C₈-Alkyl oder Halogen substituiert ist, Phenyl, Naphthyl, OR⁴, NO₂, NR³R⁴, CHF₂ und CF₃, wobei R³ und R⁴ die angegebenen Bedeutungen besitzen.

Besonders bevorzugt sind dabei die Verbindungen der Formel I, worin
- A: für SC₃-C₁₀-Alkylen, OC₃-C₁₀-Alkylen oder NR³-C₃-C₁₀-Alkylen steht, wobei R³ für H oder C₁-C₈-Alkyl steht,
- R¹: für H oder C₁-C₈-Alkyl steht;
- R²: die oben angegebenen Bedeutungen besitzt;
- B: für 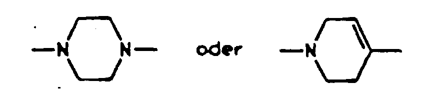 steht:
- Ar: für Phenyl steht, das ein bis vier Substituenten aufweist, die unabhängig voneinander für H, C₁-C₈-Alkyl, OC₁-C₈-Alkyl, CHF₂, CF₃ oder CN stehen.

Insbesondere weist Ar zwei Substituenten auf, die sich in 3- bzw. 5-Stellung befinden, wobei der eine Substituent CF₃, CHF₂ oder C₁-C₈-Alkyl und der andere Substituent H oder C₁-C₈-Alkyl ist.

Eine weitere bevorzugte Ausführungsform sind die Verbindungen der Formel I, worin
Ar für Pyrimidinyl steht, das ein bis drei Substituenten aufweist, die unabhängig voneinander ausgewählt sind unter H, C₁-C₈-Alkyl, Phenyl, Naphthyl, C₅-C₆-Cycloalkyl, OH, OC₁-C₈-Alkyl, Halogen, CN, CF₃, CHF₂ und einem 5- oder 6-gliedrigen, heterocyclischen, aromatischen Rest mit 1 bis 3 Heteroatomen, ausgewählt unter O, N und S;
R¹ für H oder C₁-C₈-Alkyl, das gegebenenfalls durch OH, OC₁-C₈-Alkyl oder Halogen substituiert ist, steht,
R² für H, NR³R⁴ oder OR⁴ steht, wobei R³ und R⁴ unabhängig voneinander für H, C₁-C₈-Alkyl oder Phenyl-C₁-C₈-alkyl stehen;
A für C₁-C₁₀-Alkylen steht, das gegebenenfalls wenigstens eine Gruppe umfaßt, die ausgewählt ist unter 0, S, NR³, wobei R³ für H oder C₁-C₈-Alkyl steht, und einer Doppel- oder Dreifachbindung; und
B wie oben definiert ist.

Eine weitere bevorzugte Ausführungsfcrm sind die Verbindungen der Formel I, worin
Ar für Pyridinyl steht, das ein bis vier Substituenten aufweist, die unabhängig voneinander ausgewählt sind unter H, C₁-C₈-Alkyl, Phenyl, Naphthyl, OH, OC₁-C₈-Alkyl, Haloqen, CF₃, CN, C₂-C₆-Alkenyl, C₂-C₆-Alkinyl und einem 5- oder 6-gliedrigen, heterocyclischen, aromatischen Rest mit 1 bis 3 Heteroatomen, ausgewählt unter O, N und S;
R¹ für H, C₁-C₈-Alkyl, C₃-C₆-Cycloalkyl oder OR⁴ steht, wobei R⁴ für H oder C₁-C₈-Alkyl, das gegebenenfalls durch OH, OC₁-C₈-Alkyl oder Halogen substituiert ist, steht; und
R², A und B wie oben definiert sind.

Die Erfindung umfaßt auch die Säureadditionssalze der Verbindungen der Formel I, mit physiologisch verträglichen Säuren. Als physiologisch verträgliche organische und anorganische Säuren kommen beispielsweise Salzsäure, Bromwasserstoffsäure, Phosphorsäure, Schwefelsäure, Oxalsäure, Maleinsäure, Fumarsäure, Milchsäure, Weinsäure, Adipinsäure oder Benzoesäure in Betracht. Weitere brauchbare Säuren sind in Fortschritte der Arzneimittelforschung, Band 10, Seiten 224 ff, Birkhäuser Verlag, Basel und Stuttgart, 1966, beschrieben.

Die Verbindungen der Formel I können ein oder mehrere Asymmetriezentren aufweisen. Zur Erfindung zählen daher nicht nur die Racemate, sondern auch die betreffenden Enantiomere und Diastereomere. Auch die jeweiligen tautomeren Formen zählen zur Erfindung.

Die Herstellung der Verbindungen der Formel I kann analog zu üblichen Methoden erfolgen, wie z.B. beschrieben in Houben Weyl, *"Handbuch der Organischen Chemie",* 4. Aufl. Thieme Verlag, Stuttgart 1994, Band E8/d, S.479ff; und
A.R. Katritzky, C.W. Rees (ed.), *"Comprehensive Heterocyclic Chemistry",* 1. Aufl. Pergamon Press 1984, insbesondere Vol. 5, part 4a, S. 733ff
und der dort zitierten Literatur. Das Verfahren zur Herstellung der Verbindungen besteht darin, daß man
i) eine Verbindung der allgemeinen Formel II: 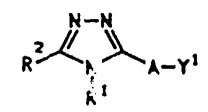 worin
   Y¹ für eine übliche Abgangsgruppe steht, mit einer Verbindung der allgemeinen Formel III

      H - B - Ar

      umsetzt;
ii) zur Herstellung einer Verbindung der Formel I,
   worin A ein Sauerstoff- oder Schwefelatom oder NR³ umfaßt:
   a) eine Verbindung der allgemeinen Formel IV: 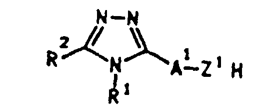 worin Z¹ für O, S oder NR³ steht und A¹ für C_{O}-C₁₈-Alkylen steht, mit einer Verbindung der allgemeinen Formel VI

      Y¹ - A² - B - Ar

      worin Y¹ die oben angegebenen Bedeutungen besitzt und A² für C₁-C₁₈-Alkylen steht, wobei A¹ und A² zusammen 1 bis 18 Kohlenstoffatome aufweisen, umsetzt;
iii) zur Herstellung einer Verbindung der Formel I, worin A die Gruppe COO oder CONR³ umfaßt:
   a) eine Verbindung der allgemeinen Formel VII: 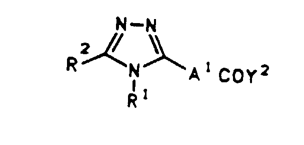 worin Y² für OH, OC₁-C₄-Alkyl, Cl oder zusammen mit CO für eine aktivierte Carboxylgruppe steht, und A¹ die oben angegebenen Bedeutungen besitzt, mit einer Verbindung der Formel VIII:

      Z¹ - A² - B - Ar

      worin A² die oben angegebenen Bedeutungen besitzt,
      und Z¹ für OH oder NHR³ steht,
      umsetzt,
iv) zur Herstellung einer Verbindung der Formel I, worin A die Gruppe OCO oder NR³CO umfaßt:
   a) eine Verbindung der Formel IV 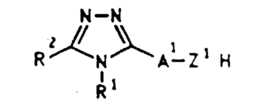 worin Z¹ für O oder NR³ steht, mit einer Verbindung der Formel X:

      Y²CO - A² - B - Ar

      worin B und Y² die oben angegebenen Bedeutungen besitzen, umsetzt, wobei R¹, R², A, B und Ar die oben angegebenen Bedeutungen besitzen.

Die oben beschriebenen Umsetzungen erfolgen im allgemeinen in einem Lösungsmittel bei Temperaturen zwischen Raumtemperatur und der Siedetemperatur des verwendeten Lösungsmittels. Brauchbare Lösungsmittel sind beispielsweise Ethylacetat, Tetrahydrofuran, Dimethylformamid, Dimethoxyethan, Toluol, Xylol oder ein Keton, wie Aceton oder Methylethylketon.

Gewünschtenfalls arbeitet man in Gegenwart eines säurebindenden Mittels. Geeignete säurebindende Mittel sind anorganische Basen, wie Natrium- oder Kaliumcarbonat, Natriummethylat, Natriumethylat, Natriumhydrid oder organische Basen, wie Triethylamin oder Pyridin. Letztere können gleichzeitig als Lösungsmittel dienen.

Die Isolierung des Rohprodukts erfolgt in üblicher Weise, beispielsweise durch Filtration, Abdestillieren des Lösungsmittels oder Extraktion aus dem Reaktionsgemisch etc. Die Reinigung der erhaltenen Verbindung kann in üblicher Weise erfolgen, beispielsweise durch Umkristallisieren aus einem Lösungsmittel, Chromatographie oder Überführen in eine Säureadditionsverbindung.

Die Säureadditionssalze werden in üblicher Weise durch Mischen der freien Base mit der entsprechenden Säure, gegebenenfalls in Lösung in einem organischen Lösungsmittel, beispielweise einem niedrigen Alkohol, wie Methanol, Ethanol oder Propanol, einem Ether, wie Methyl-t-butylether, einem Keton, wie Aceton oder Methylethylketon oder einem Ester, wie Essigsäureethylester, hergestellt.

Die oben erwähnten Ausgangsmaterialien sind literaturbekannt oder können nach bekannten Verfahren hergestellt werden.

Zur Behandlung der oben erwähnten Erkrankungen werden die erfindungsgemäßen Verbindungen in üblicher Weise oral oder parenteral (subkutan, intravenös, intramuskulär, intraperitoneal) verabreicht. Die Applikation kann auch mit Dämpfen oder Sprays durch den Nasen-Rachen-Raum erfolgen.

Die Dosierung hängt vom Alter, Zustand und Gewicht des Patienten sowie von der Applikationsart ab. In der Regel beträgt die tägliche Wirkstoffdosis etwa 10 bis 1000 mg pro Patient und Tag bei oraler Gabe und etwa 1 bis 500 mg pro Patient und Tag bei parenteraler Gabe.

Die Erfindung betrifft auch pharmazeutische Mittel, die die erfindungsgemäßen Verbindungen enthalten. Diese Mittel liegen in den üblichen galenischen Applikationsformen in fester oder flüssiger Form vor, beispielsweise als Tabletten, Filmtabletten, Kapseln, Pulver, Granulate, Dragees, Suppositorien, Lösungen oder Sprays. Die Wirkstoffe können dabei mit den üblichen galenischen Hilfsmitteln, wie Tablettenbindemitteln, Füllstoffen, Konservierungsmitteln, Tablettensprengmitteln, Fließregulierungsmitteln, Weichmachern, Netzmitteln, Dispergiermitteln, Emulgatoren, Lösungsmitteln, Retardierungsmitteln, Antioxidantien und/oder Treibgasen verarbeitet werden (vgl. H. Sucker et al., Pharmazeutische Technologie, Thieme-Verlag, Stuttgart, 1978). Die so erhaltenen Applikationsformen enthalten den Wirkstoff normalerweise in einer Menge von 1 bis 99 Gew.-%.

Die nachfolgenden Beispiele dienen zur Erläuterung der Erfindung, ohne sie zu begrenzen.

### Beispiel 1

### 4-Methyl-3-[3-(4-{3-trifluormethylphenyl}piperazinyl)-propylmercapto]-4H-1,2,4-triazol

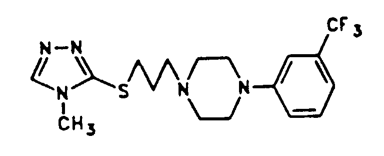

a) 1-(3-Chlorpropyl)-4-(3-trifluormethylphenyl)piperazin
   30 g (0,13 mol) m-Trifluormethylphenylpiperazin, 23 g (0,146 mol) 1,3-Bromchlorpropan und 15 g (0,148 mol) Triethylamin wurden in 200 ml THF 4 Stunden unter Rückfluß erhitzt. Nach Abkühlen wurde abgesaugt und eingeengt. Der zähflüssige Rückstand wurde mit Essigester aufgenommen, mit Wasser gewaschen, über MgSO₄ getrocknet und anschließend eingeengt. Als Rückstand erhielt man 39 g Produkt als gelbliches Öl (quantitative Ausbeute).
b) 4-Methyl-3-[3-(4-{3-trifluormethylphenyl}piperazinyl)-propylmercapto]-4H-1,2,4-triazol
   1,15 g (10 mmol) 3 Mercapto-4-methyl-4H-1,2,4-triazol, 3,1 g (10,1 mmol) 1-(3-Chlorpropyl)-4-(3-trifluormethylphenyl)piperazin und 1,5 g (15 mmol) Triethylamin wurden in 5 ml DMF 1 Stunde bei 100°C gerührt. Anschließend wurde auf 5%ige Salzsäure gegossen und mit Essigester extrahiert. Nach Alkalisieren der wäßrigen Phase mit Natronlauge wurde wieder mit Essigester extrahiert, die organische Phase über MgSO₄ getrocknet und eingeengt. Der Rückstand wurde chromatographisch gereinigt (Laufmittel : CH₂Cl₂/CH₃OH = 95/5). Es wurden 2,1 g Produkt als gelbliches Öl erhalten ( = 55 % Ausbeute)
   - H-NMR [δ,ppm]:: 2,02(2H); 2,55(2H); 2,61(4H); 3,23(6H);3,33(2H);3,61(3H); 7,06(3H);7,33(1H);8,12(1H)

### Beispiel 2

### 4-Methyl-3-[5-(4-{3-trifluormethylphenyl}piperazinyl)-pentylmercapto]-4H-1,2,4-triazol

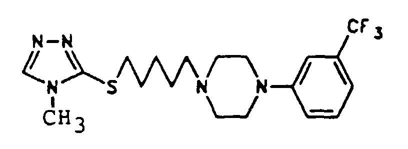

a) 3-(5-Chlorpentylmercapto)-4-methyl-4H-1,2,4-triazol
   2,88 g (25 mmol) 3-Mercapto-4-methyl-4H-1,2,4-triazol, 4,64 g (25 mmol) 1,5-Bromchlorpentan und 5,58 g (25,5 mmol) Triethylamin wurden in 100 ml THF 4 Stunden unter Rückfluß erhitzt. Nach dem Abkühlen wurde abgesaugt, eingeengt und der Rückstand chromatographisch gereinigt (Laufmittel : CH₂Cl₂/CH₃OH = 95/5). Man erhielt 1,9 g Produkt (= 35 % Ausbeute).
b) 4-Methyl-3-[5-(4-{3-trifluormethylphenyl}piperazinyl)-pentylmercapto]-4H-1,2,4-triazol
   1,9 g (8,66 mmol) Produkt aus 2a), 2,19 g (9,52 mmol) m-Trifluormethylphenylpiperazin und 0,96 g (9,52 mmol) Triethylamin wurden in 5 ml DMF 5 Stunden bei 90°C gerührt. Anschließend wurde auf Wasser gegossen und dreimal mit CH₂Cl₂ extrahiert, über MgSO₄ getrocknet und eingeengt. Der Rückstand wurde mit Methyl-t-butylether versetzt, abgesaugt und die Mutterlauge eingeengt. Nach chromatographischer Reinigung (Laufmittel : CH₂Cl₂/CH₃OH = 95/5) erhielt man 2,1 g Produkt ( = 59 % Ausbeute)
   Smp. 70 - 76°C

Analog wurden folgende Verbindungen hergestellt:

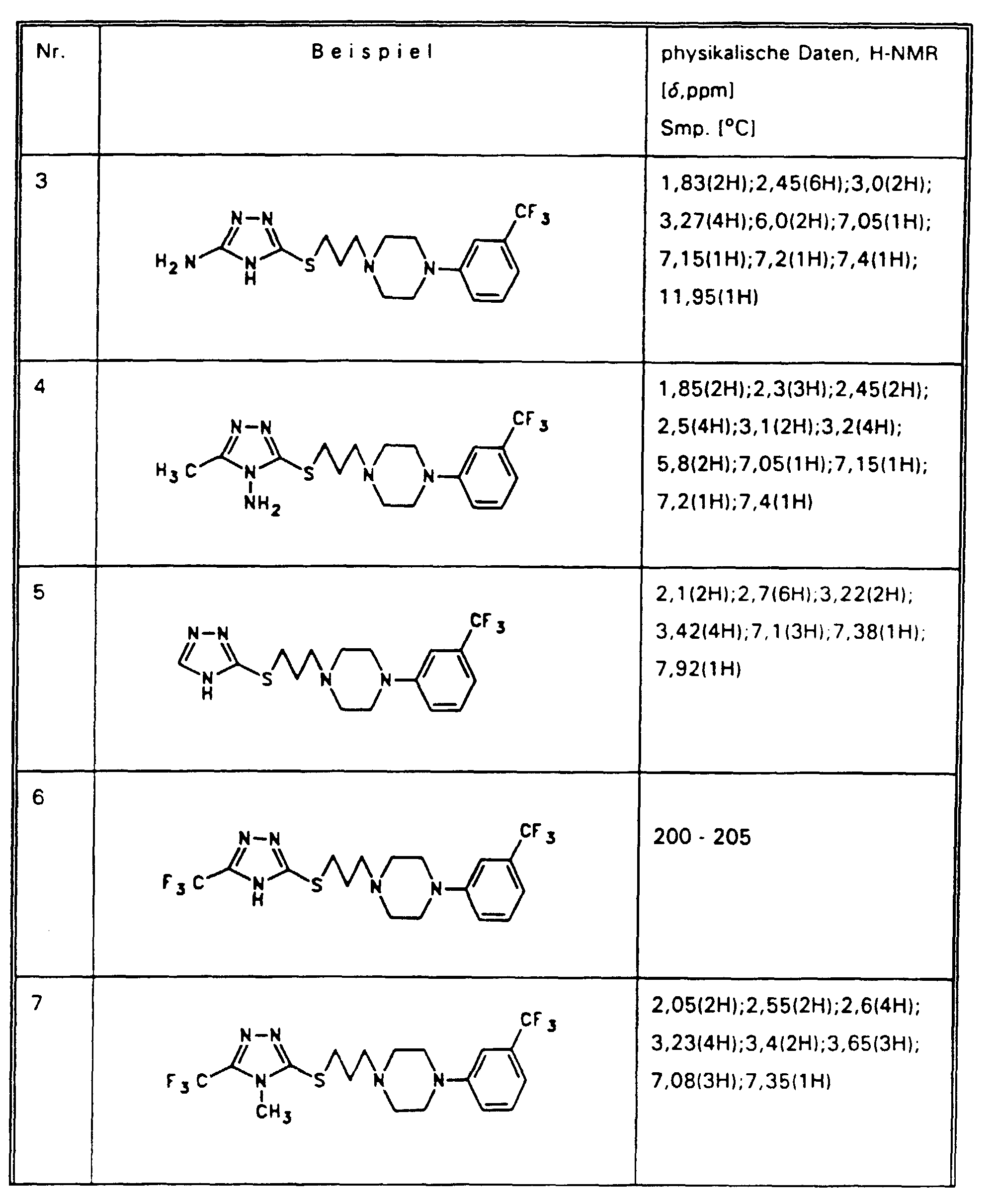

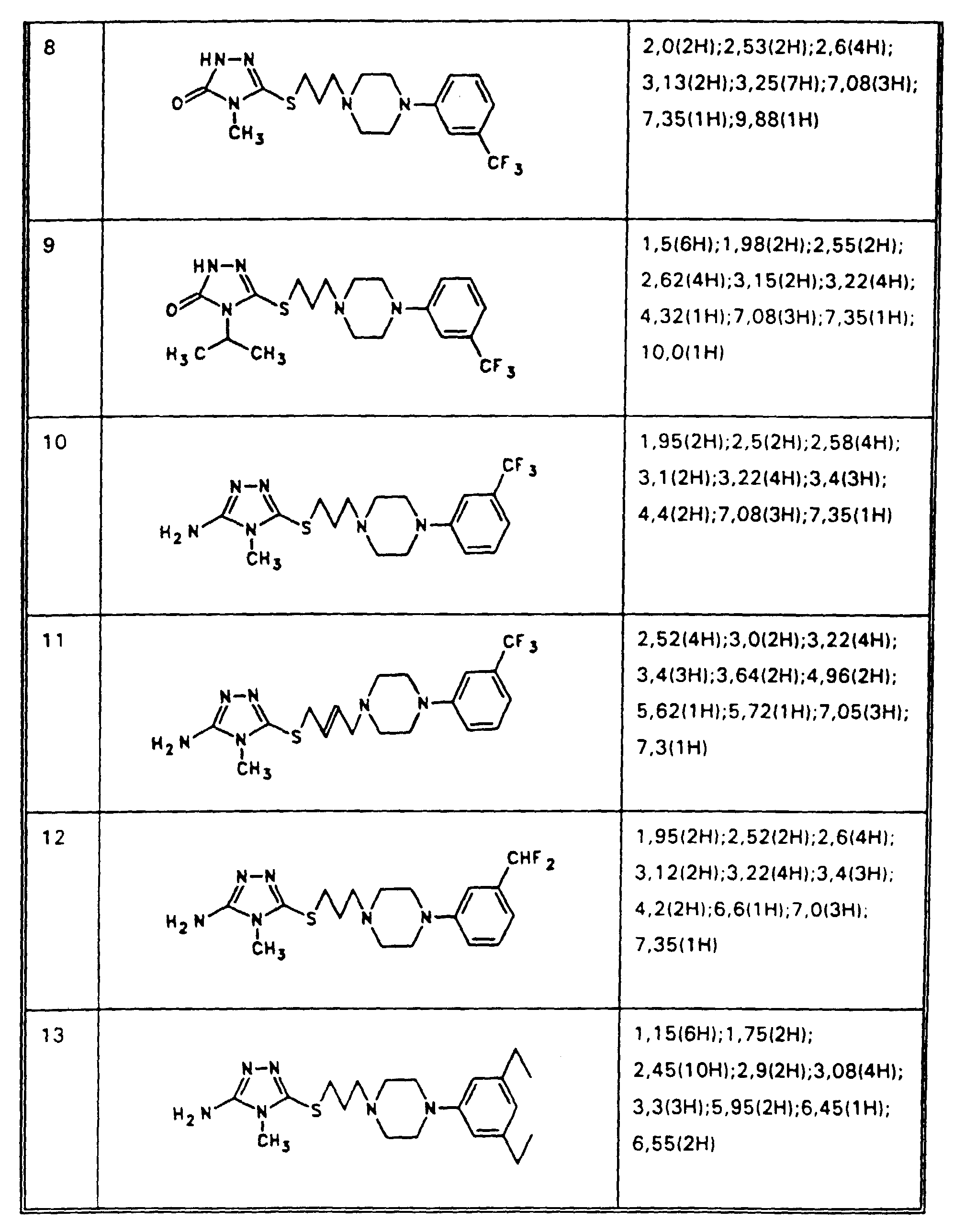

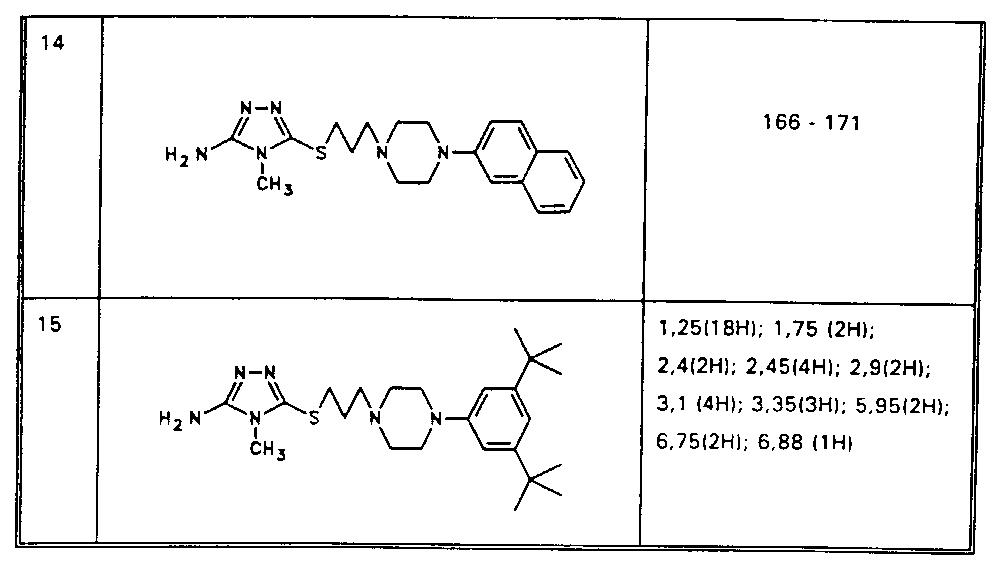

In analoger Weise wurden die in den nachfolgenden Tabellen 1 bis 3 zusammengestellten erfindungsgemäßen Verbindungen erhalten.

Die in den nachfolgenden Tabellen 4 bis 8 zusammengestellten Verbindungen können ebenfalls in analoger Weise erhalten werden.

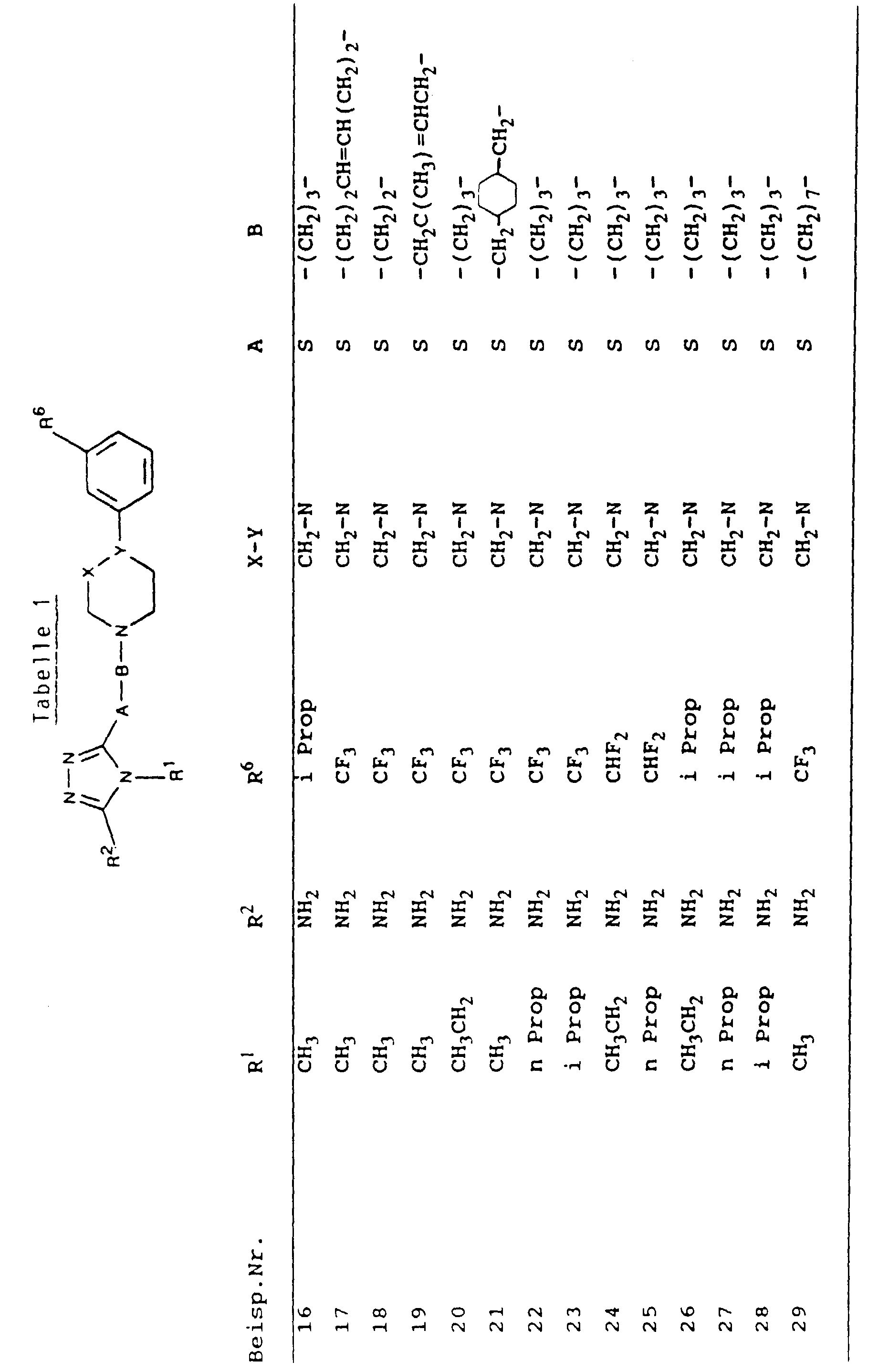

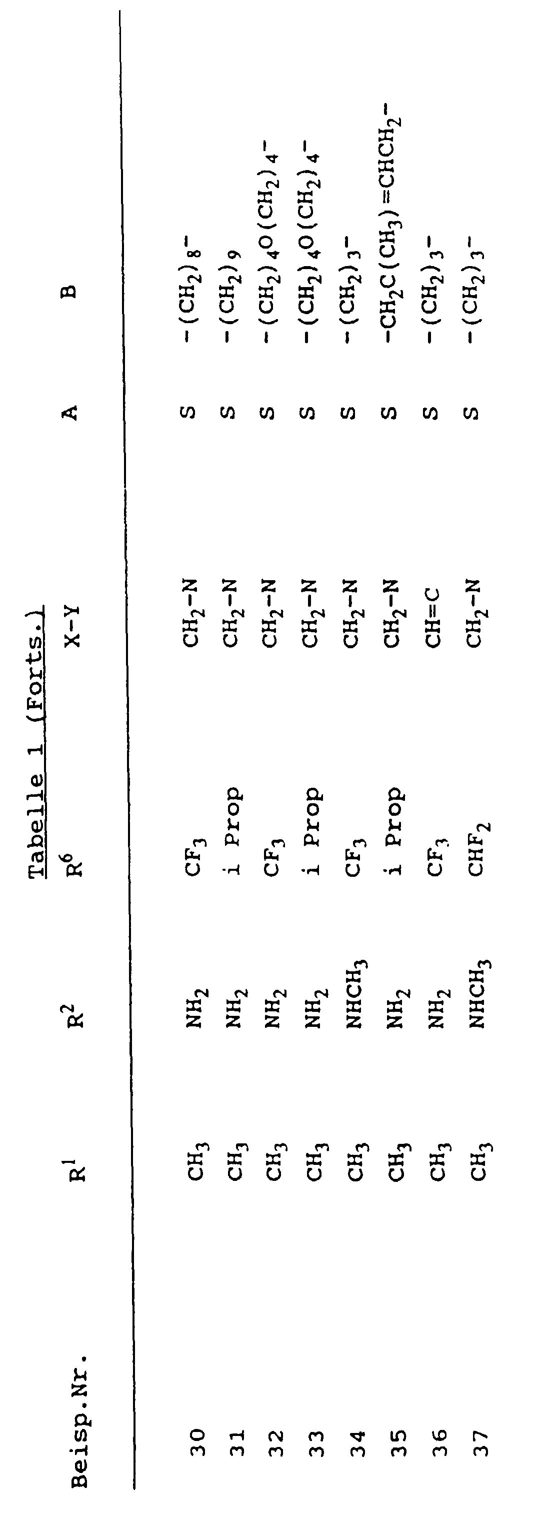

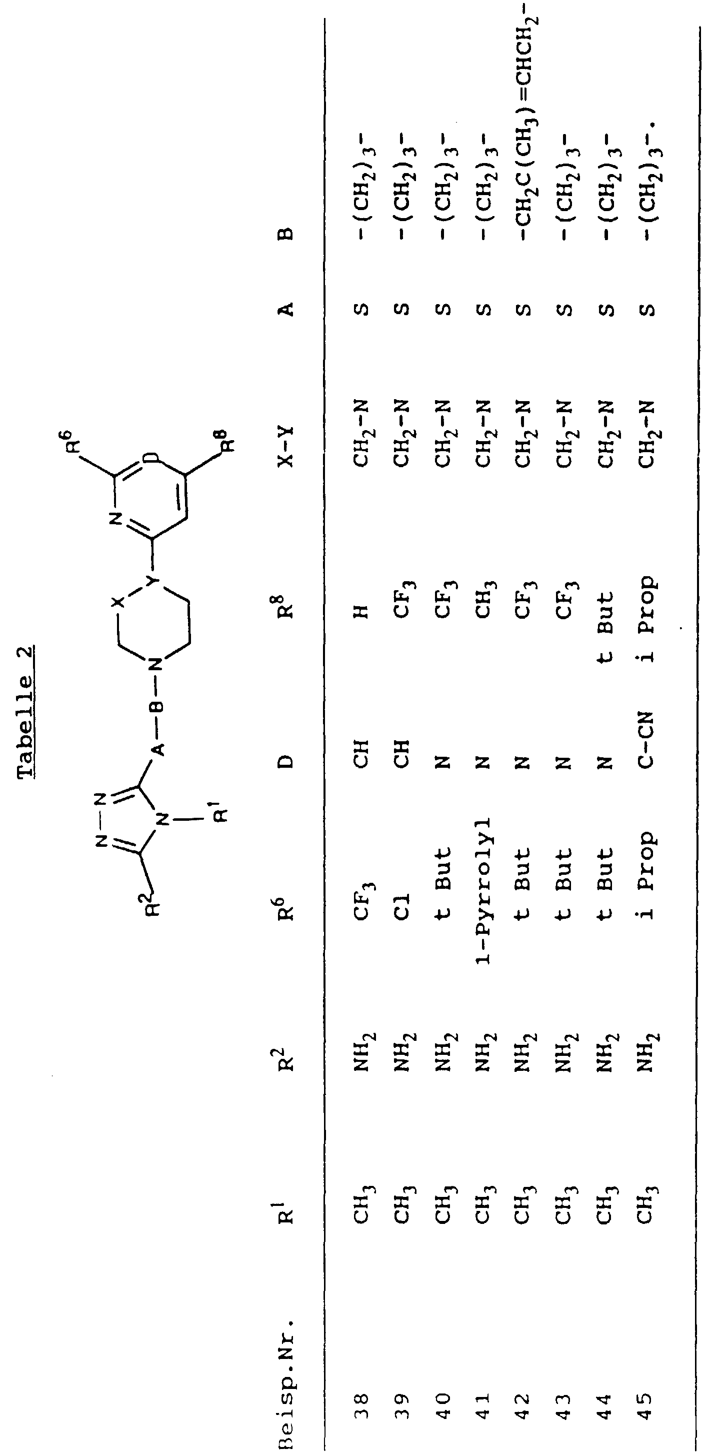

**Tabelle 3**

| Physikalische Daten der Verbindungen der Beispiele 16-45 | | |
|---|---|---|
| Beisp. Nr. | Schmp.°C | ¹H-NMR |
| 16 | | 1,2 (6H); 1,9 (2H); 2,5 (6H); 2,8 (1H); 3,2 (6H); 3,5 (3H); 4,4 (2H); 6,7 (3H); 7,1 (1H) |
| 17 | 194-196° Dihydrochlorid | |
| 18 | 109-110° Hydrochlorid | |
| 19 | 132-134° | |
| 20 | | 1,3 (3H); 2,0 (2H); 2,5 (6H); 3,2 (6H); 3,8 (2H; 4,6 (2H); 7,0 (3H); 7,4 (1H) |
| 21 | 154-155° | |
| 22 | | 1,0 (3H); 1,8 (2H); 2,0 (2H); 2,5 (6H); 3,1 (6H); 3,7 (2H); 4,4 (2H); 7,0 (3H); 7,3 (1H) |
| 23 | | 1,2 (6H); 2,0 (2H); 2,3 (6H); 3,1 (6H); 4,1 (2H); 4,3 (1H); 7,0 (3H); 7,2 (1H) |
| 24 | | 1,2 (3H); 1,8 (2H); 2,4 (2H) 2,5 (4H); 2,9 (2H); 3,1 (4H); 3,8 (2H); 6,0 (2H); 6,9 (lH); 7,0 (3H), 7,3 (1H) |
| 25 | | 1,0 (3H); 1,7 (2H); 2,0 (2H); 2,5 (2H); 2,6 (4H); 3,0 (6H), 3,7 (2H), 4,6 (2H); 6,6 (1H); 7,0 (3H); 7,4 (1H) |
| 26 | | 1,2 (9H); 1,9 (2H); 2,5 (2H); 2,6 (4H); 2,9 (1H); 3,15 (6H); 3,8 (2H); 6,8 (3H); 7,2 (1H) |
| 27 | | 0,9 (3H); 1,2 (6H), 1,7 (2H); 1,9 (2H); 2,5 (2H); 2,6 (4H); 2,8 (1H); 2,9 (2H); 3,2 (4H); 3,4 (2H); 6,8 (3H); 7,3 (1H) |
| 28 | | 1,2 (6H); 1,5 (6H); 1,9 (2H); 2,4 (2H); 2,5 (4H); 2,8 (1H); 3,2 (6H), 4,3 (3H); 6,75 (3H), 7,15 (1H) |
| 29 | 118-119° | |
| 30 | 164-166° Fumarat | |
| 31 | | 1,2 (6H); 1,4 (14H), 1,7 (2H); 2,4 (2H), 2,6 (4H), 2,8 (1H); 3,0 (2H); 3,2 (4H), 3,4 (3H), 4,6 (2H), 6,8 (3H); 7,2 (1H) |
| 32 | | 1,7 (8H); 2,4 (2H); 2,6 (4H); 3,0 (2H; 3,3 (4H); 3,5 (7H); 4,8 (2H); 7,1 (3H); 7,3 (1H) |
| 33 | | 1,2 (6H); 1,6 (8H); 2,4 (2H); K 2,6 (4H); 2,9 (1H);3,1 (2H); 3,2 (4H); 3,3 (7H); 4,8 (2H); 6,8 (3H); 7,2 (1H) |
| 34 | 234-270° Trihydrochlorid | |
| 35 | 126-129° | |
| 36 | 93-100° | |
| 37 | 234-235° Dihydrochorid | |
| 38 | 153-155° | |
| 39 | 116-118° | |
| 40 | 51-60° | |
| 41 | 65-67° | |
| 42 | 67-72° | |
| 43 | 121-126° | |
| 44 | 180-183° Fumarat | |
| 45 | 130-133° | |

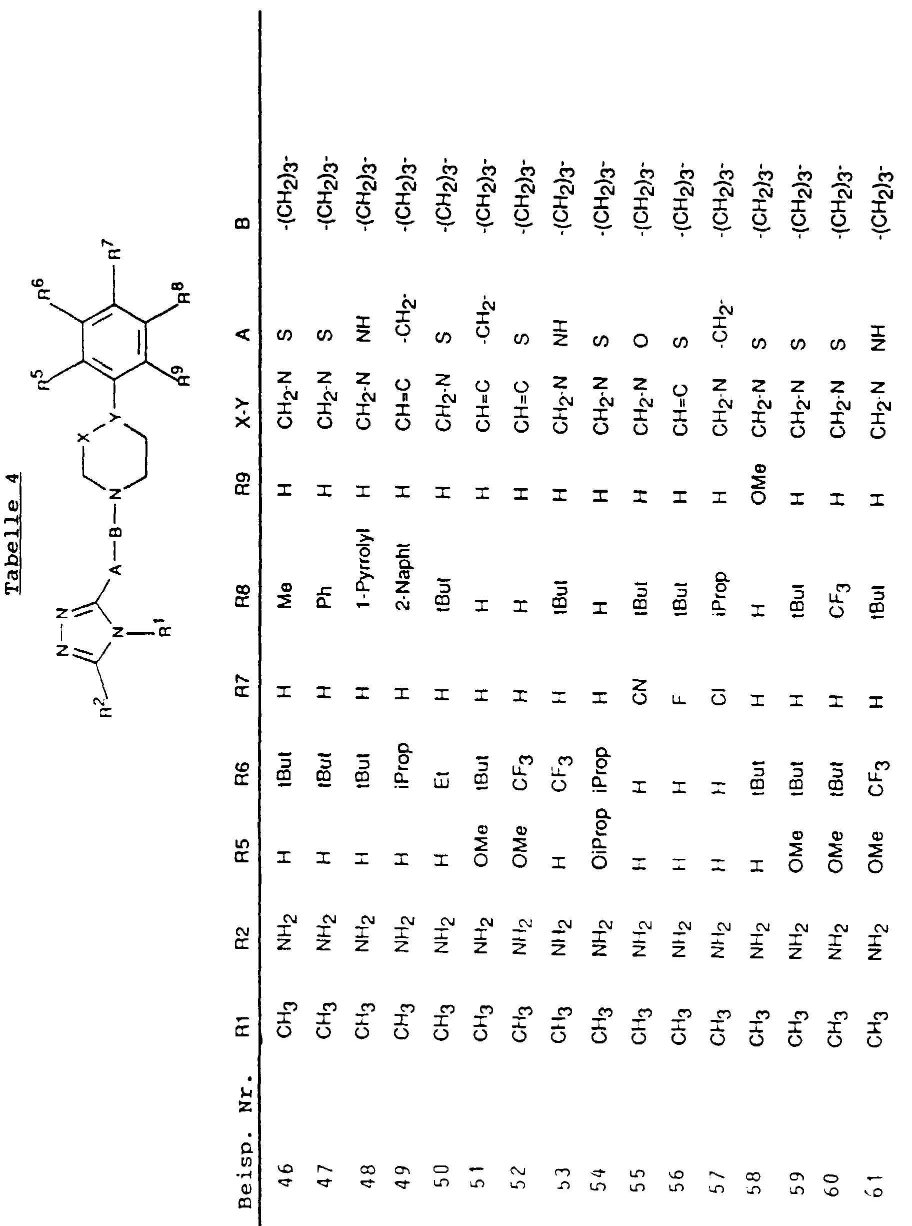

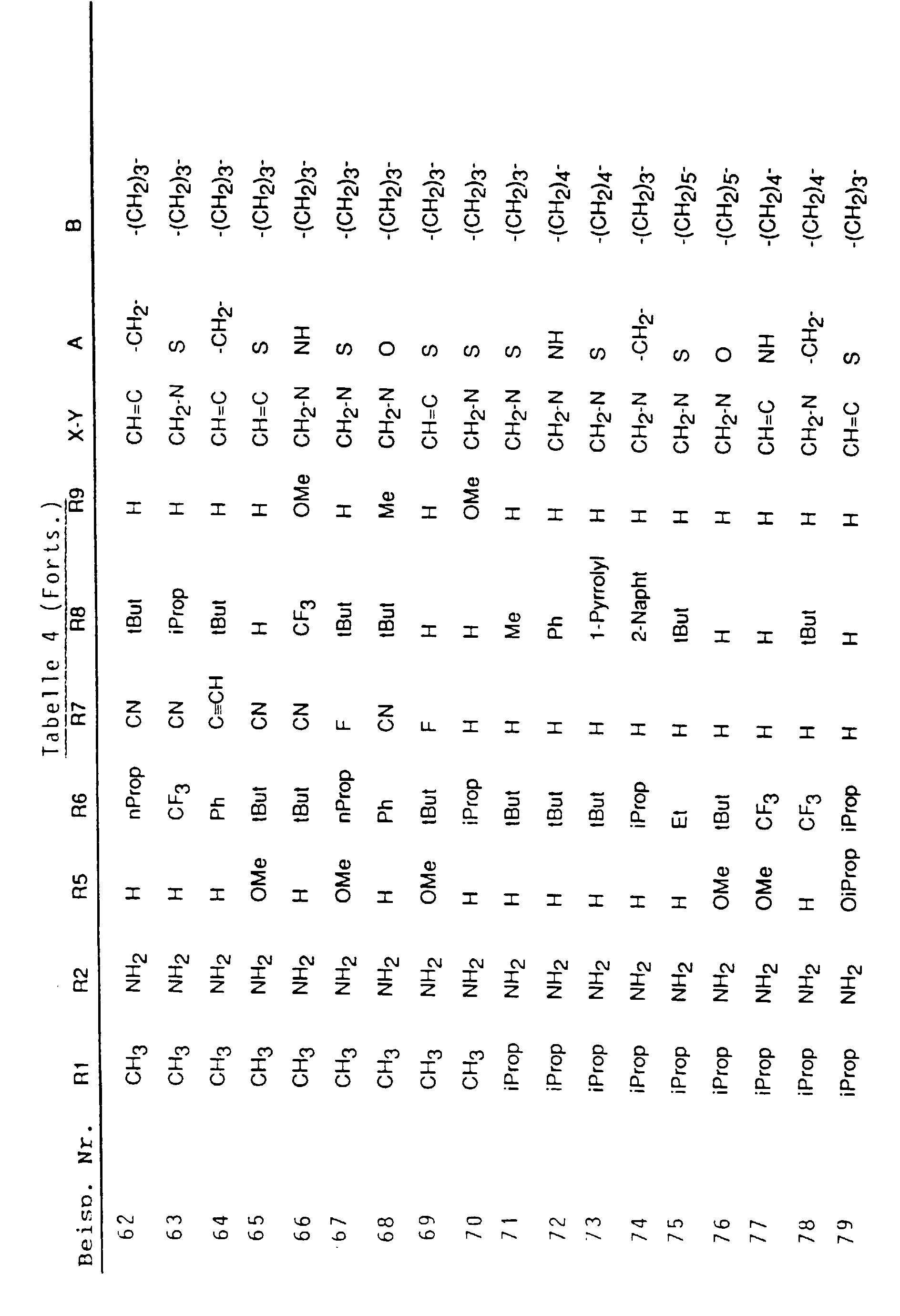

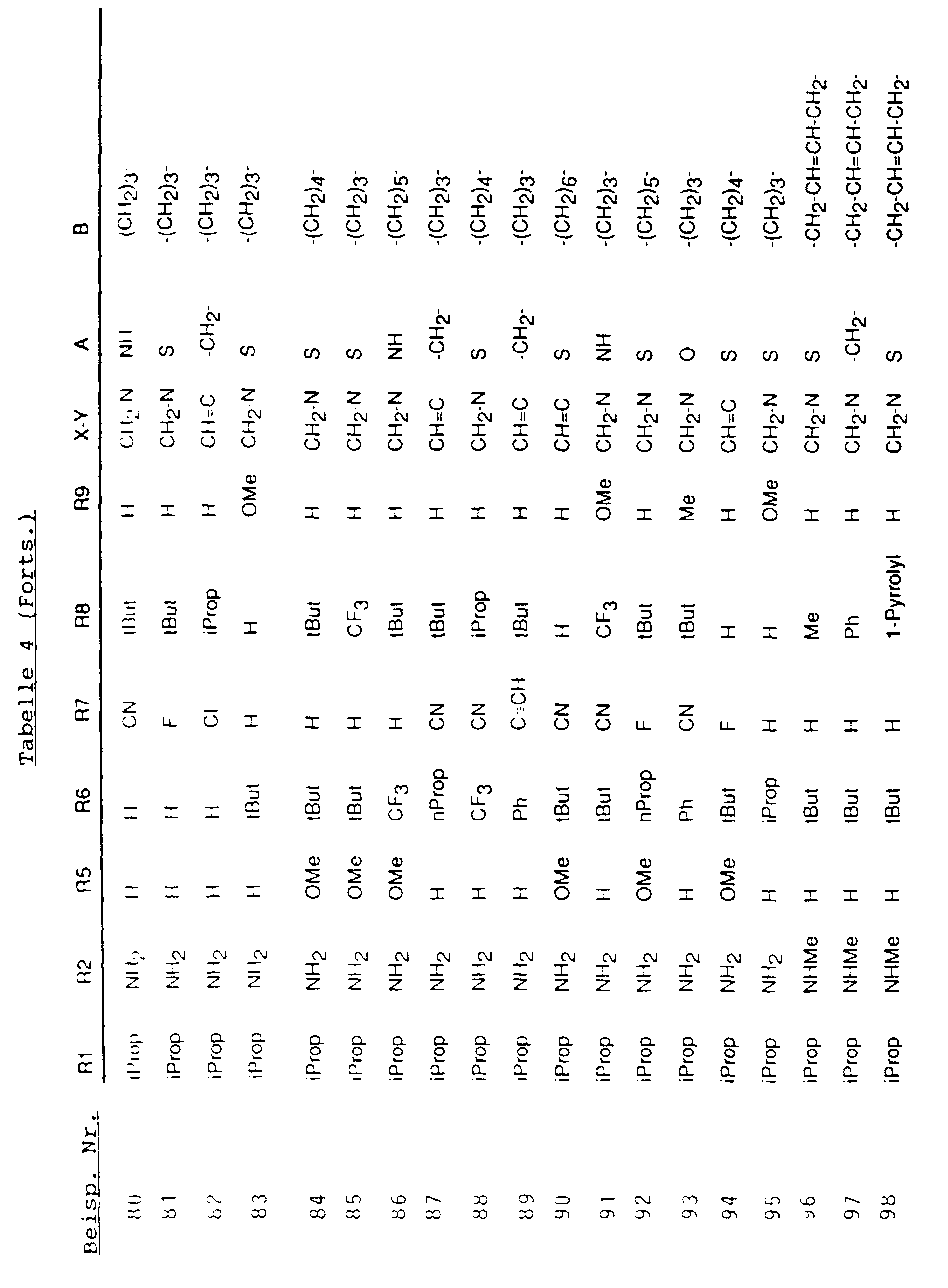

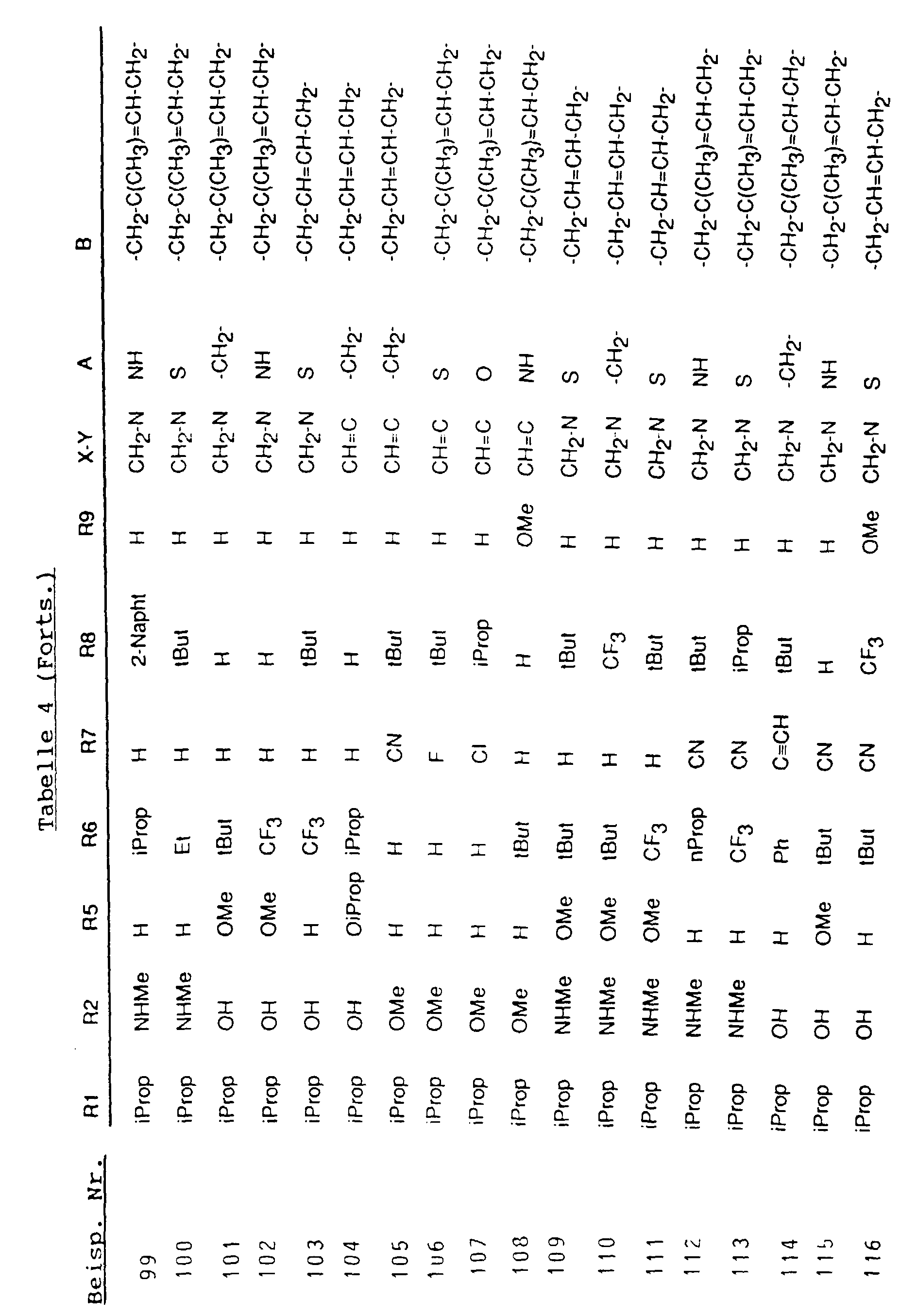

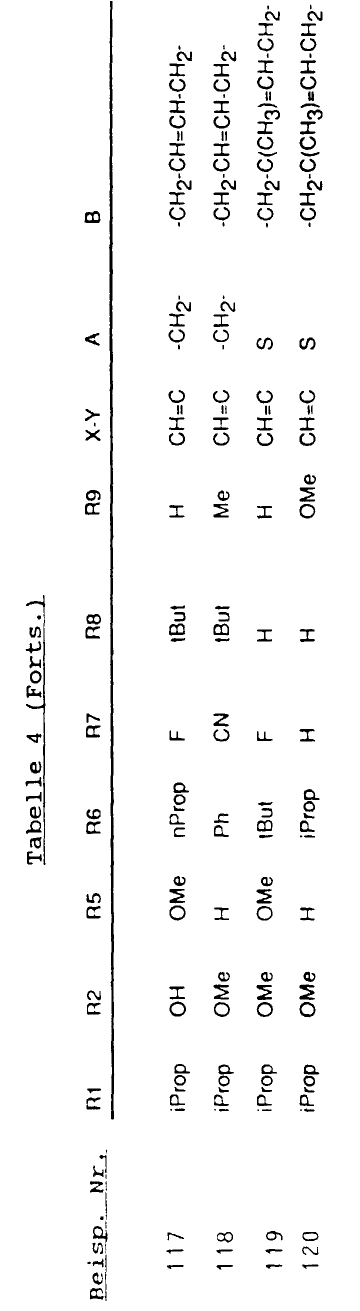

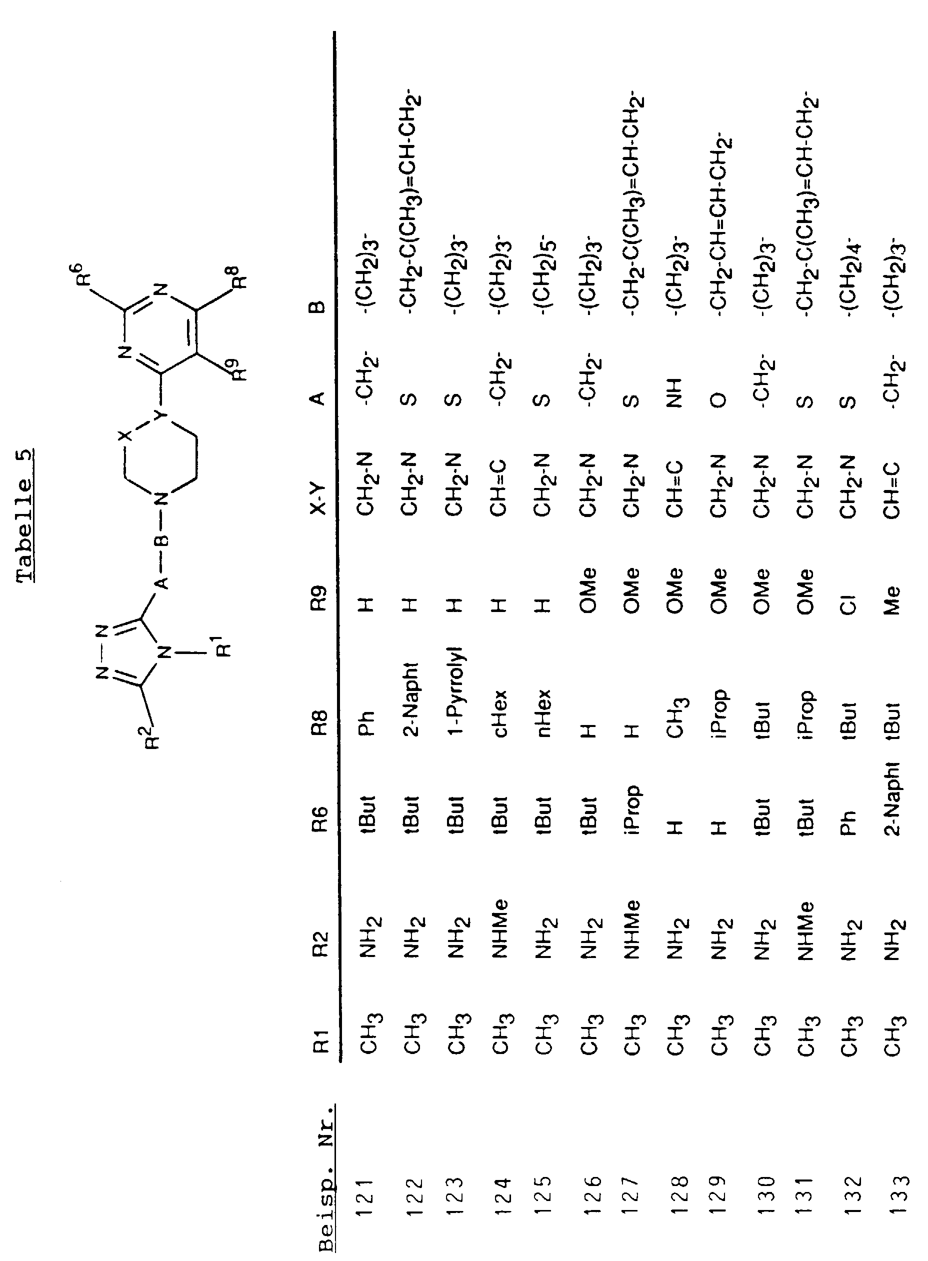

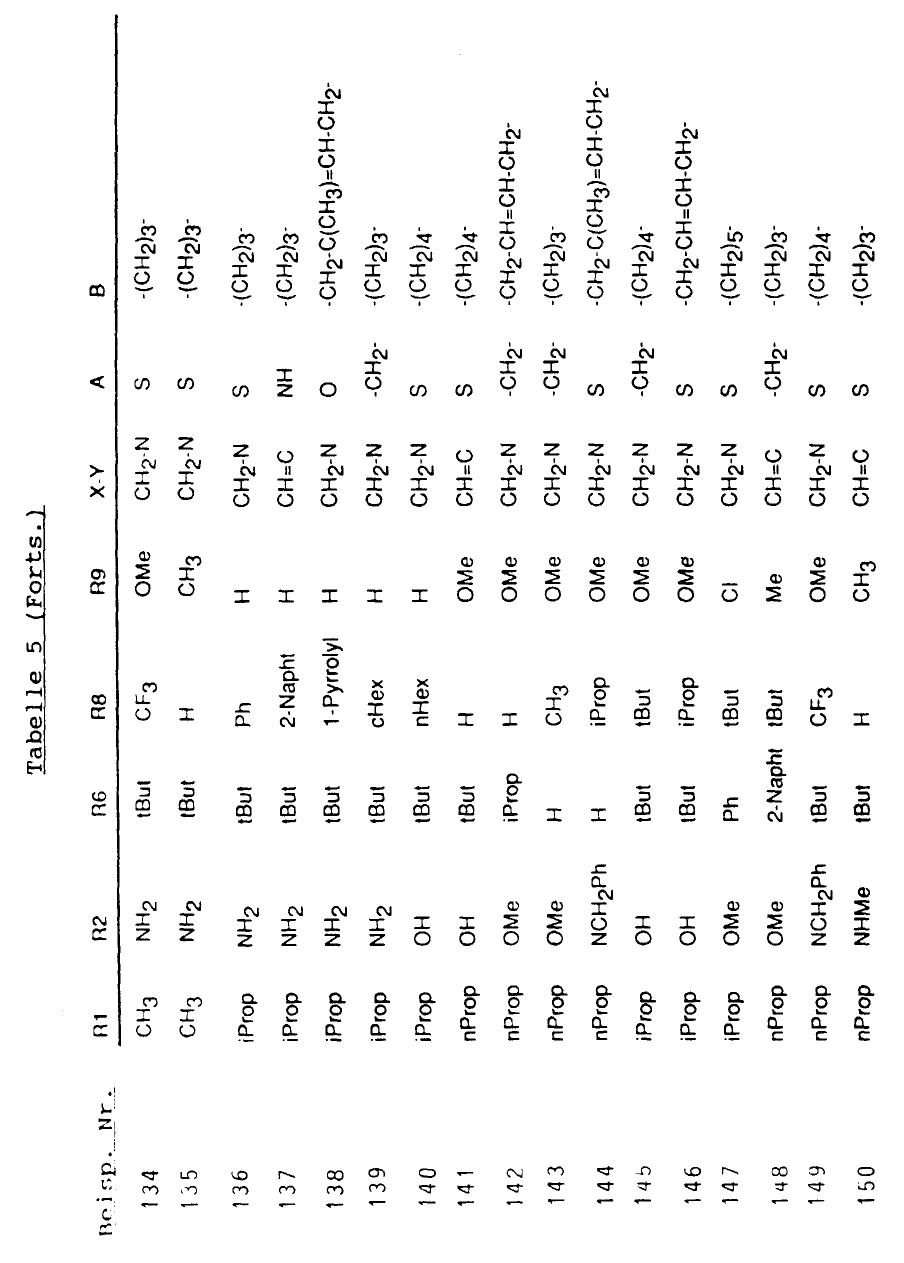

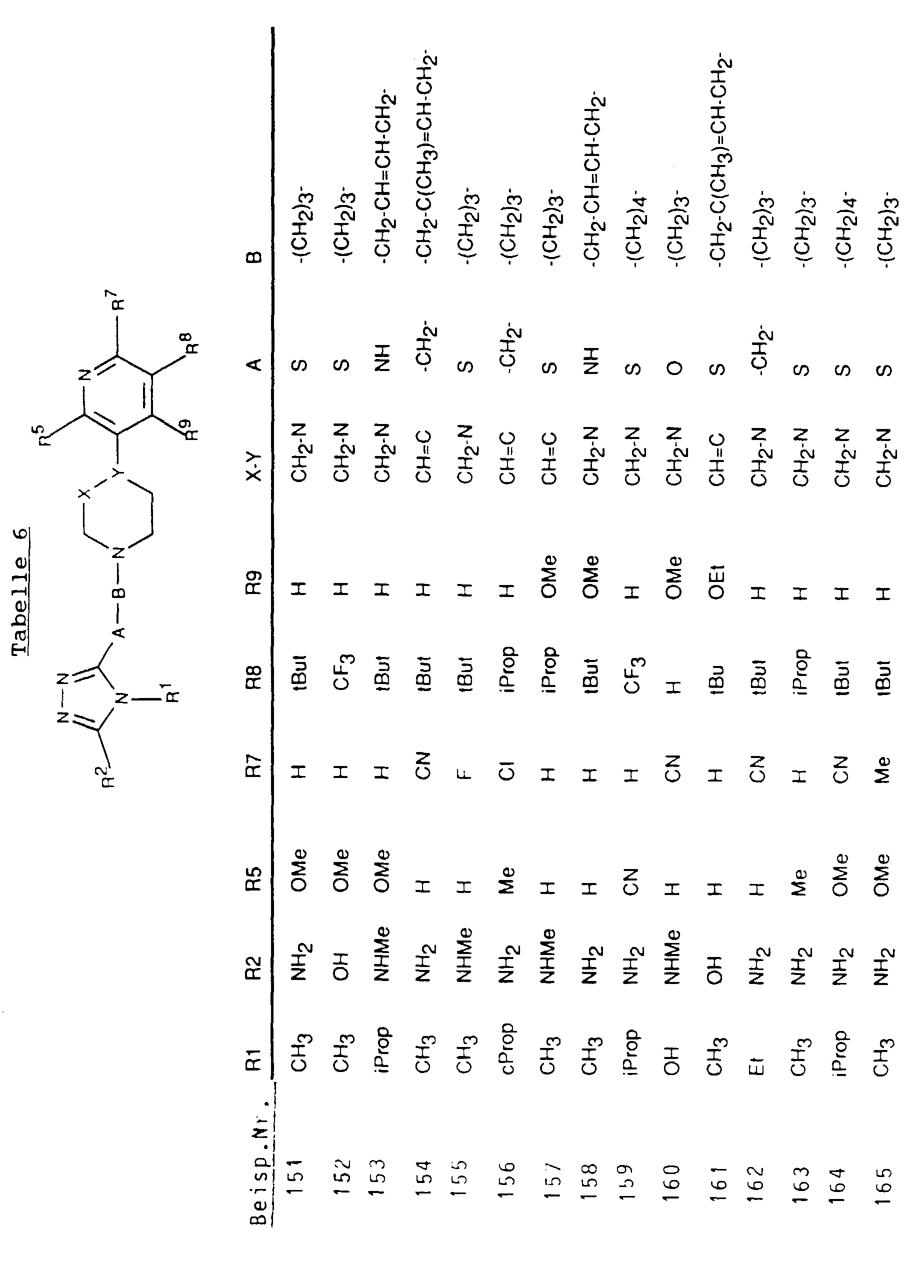

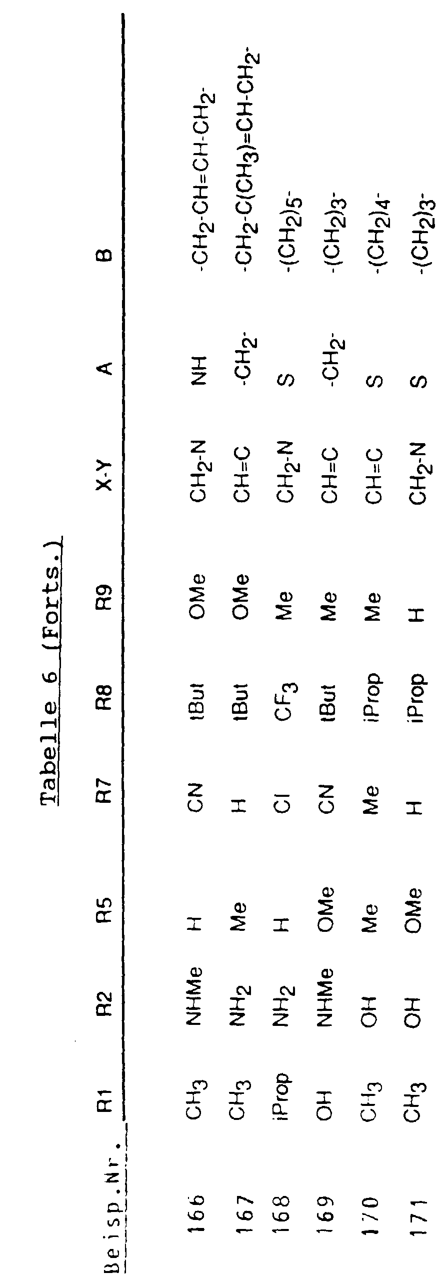

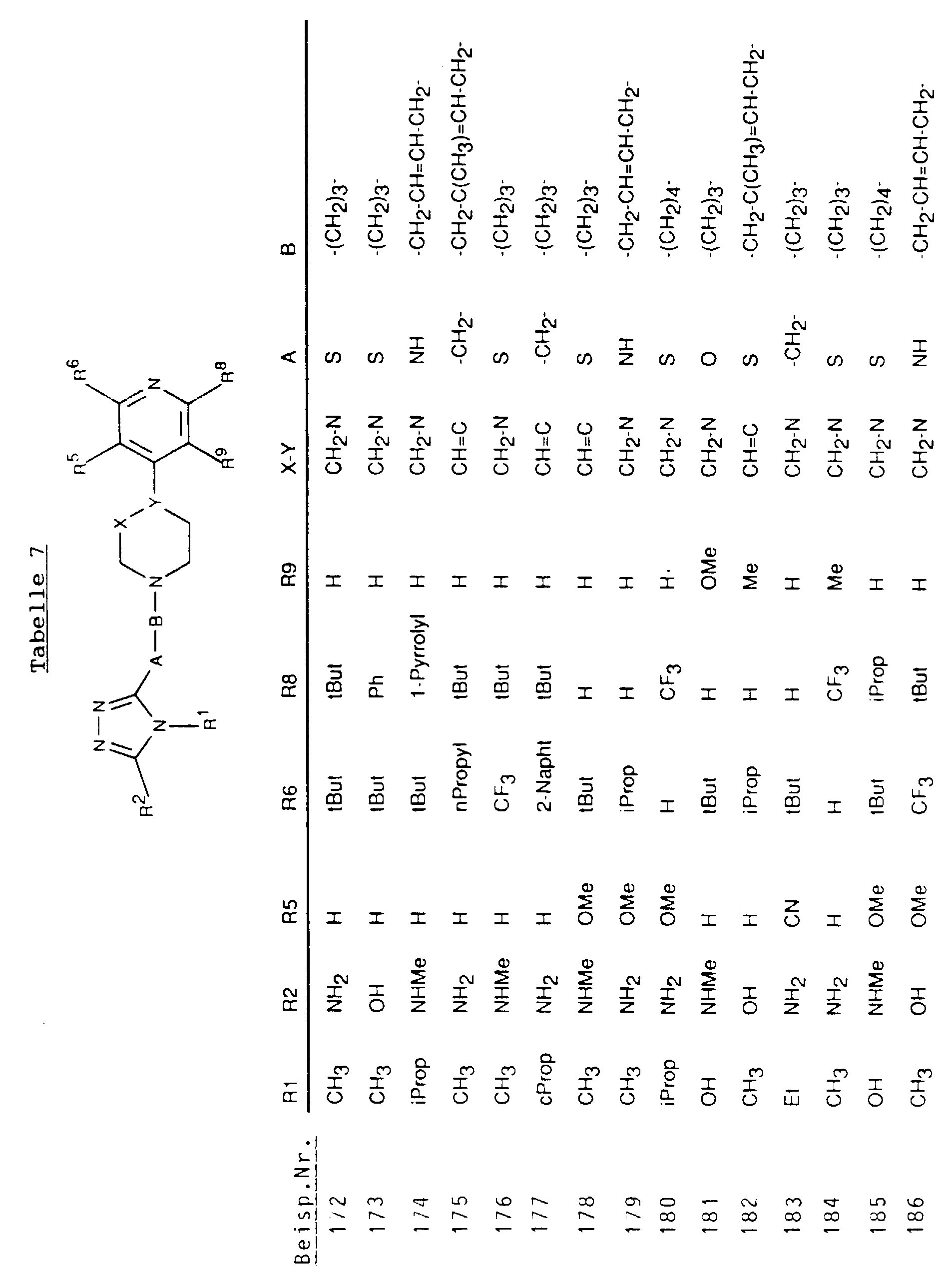

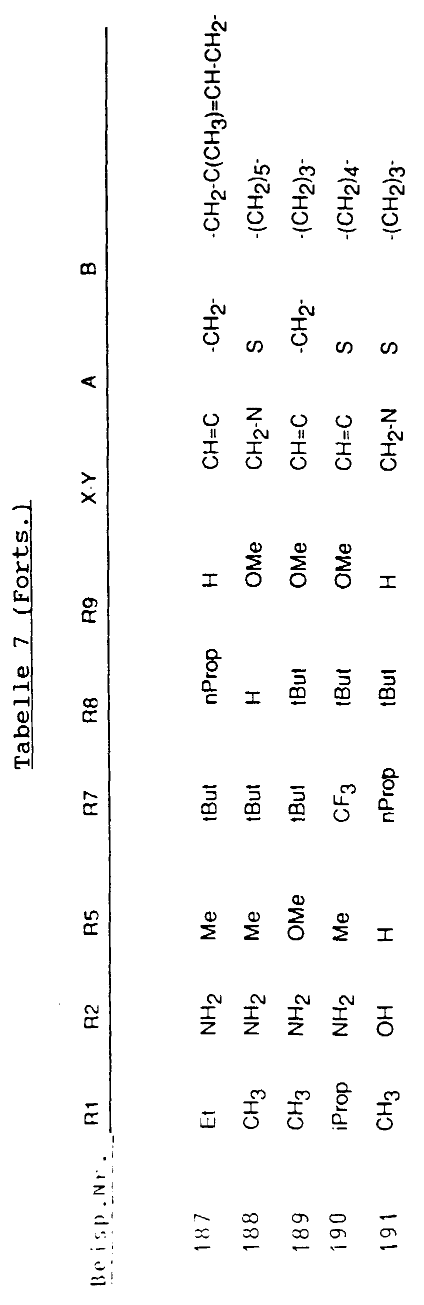

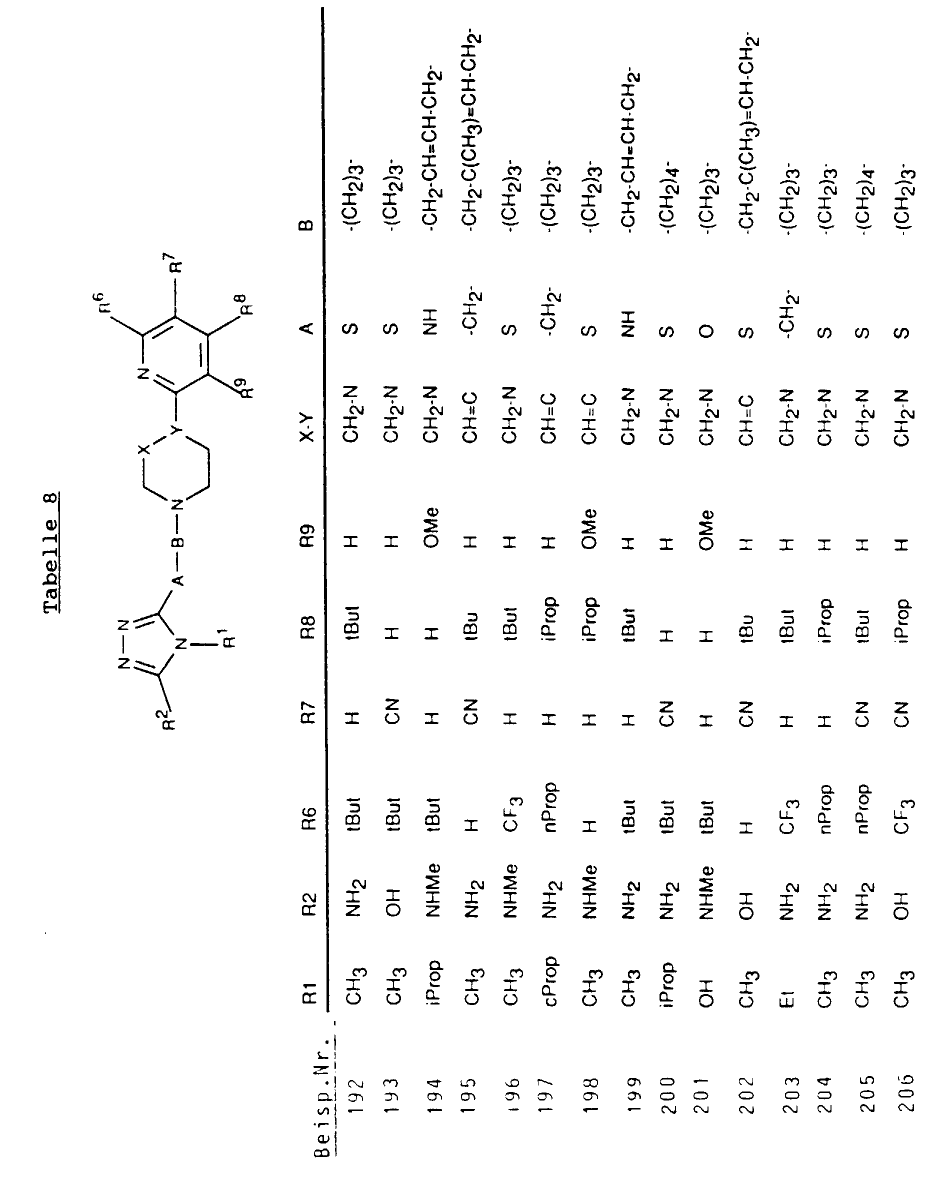

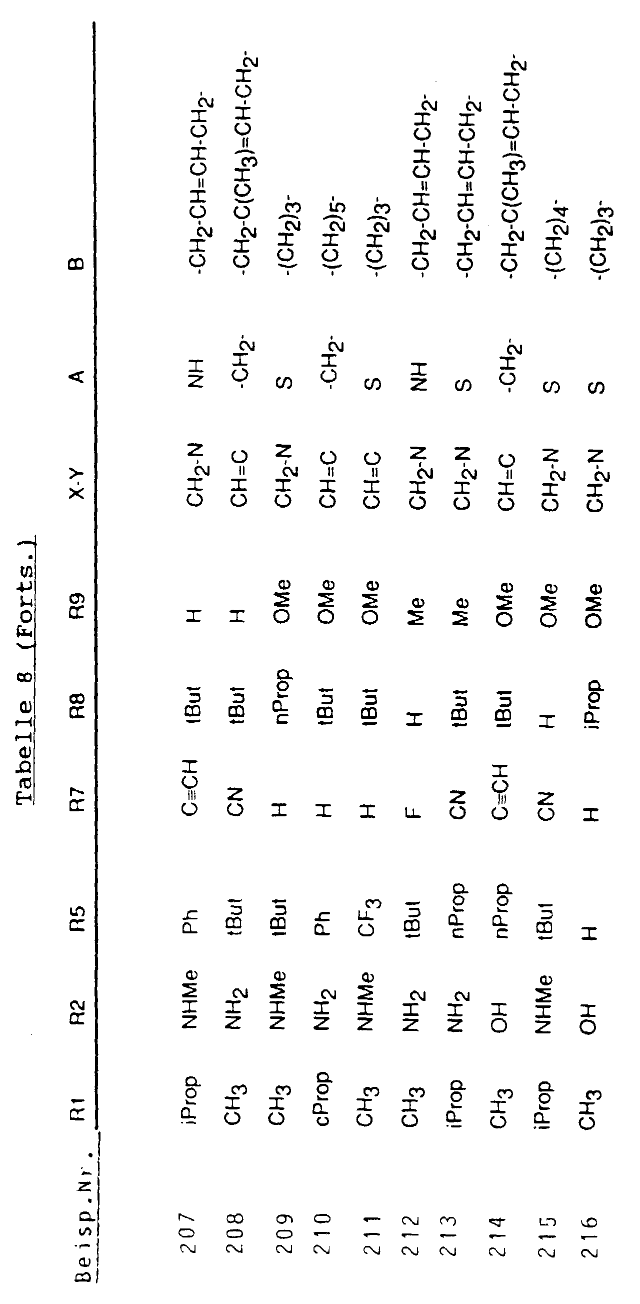

### Beispiele für galenische Applikationsformen:

### A) Tabletten

Auf einer Tablettenpresse werden in üblicher Weise Tabletten folgender Zusammensetzung gepreßt:

| | |
|---|---|
| 40 mg | Substanz des Beispiels 1 |
| 120 mg | Maisstärke |
| 13,5 mg | Gelatine |
| 45 mg | Milchzucker |
| 2,25 mg | Aerosil@ (chemisch reine Kieselsäure in submikroskopisch feiner Verteilung) |
| | |
| 6,75 mg | Kartoffelstärke (als 6 %iger Kleister) |

### B) Dragees

| | |
|---|---|
| 20 mg | Substanz des Beispiels 4 |
| 60 mg | Kernmasse |
| 70 mg | Verzuckerungsmasse |

Die Kernmasse besteht aus 9 Teilen Maisstärke, 3 Teilen Milchzucker und 1 Teil Vinylpyrrolidon-Vinylacetat-Mischpolymerisat 60:40. Die Verzuckerungsmasse besteht aus 5 Teilen Rohrzucker, Teilen Maisstärke, 2 Teilen Calciumcarbonat und 1 Teil Talk. Die so hergestellten Dragees werden anschließend mit einem magensaftresistenten Überzug versehen.

### Biologische Untersuchungen - Rezeptorbindungsstudien

### 1) D₃-Bindungstest

Für die Bindungsstudien wurden klonierte humane D₃-Rezeptor-exprimierende CCL 1,3 Mäusefibroblasten, erhältlich bei Res. Biochemicals Internat. One Strathmore Rd., Natick, MA 01760-2418 USA, eingesetzt.

### Zellpräparation

Die D₃ exprimierenden Zellen wurden in RPMI-1640 mit 10 % fötalem Kälberserum (GIBCO Nr. 041-32400 N); 100 E/ml Penicillin und 0,2 % Streptomycin (GIBCO BRL, Gaithersburg, MD, USA) vermehrt. Nach 48 h wurden die Zellen mit PBS gewaschen und mit 0,05 % trypsinhaltiger PBS 5 min inkubiert. Danach wurde mit Medium neutralisiert und die Zellen durch Zentrifugation bei 300 xg gesammelt. Zur Lyse der Zellen wurde kurz das Pellet mit Lysispuffer (5mM Tris-HCl, pH 7,4 mit 10 % Glycerin) gewaschen und danach in einer Konzentration von 10⁷-Zellen /ml Lysispuffer 30 min bei 4°C inkubiert. Die Zellen wurden bei 200 xg 10 min zentrifugiert und das Pellet in flüssigem Stickstoff gelagert.

### Bindungstests

Für den D₃-Rezeptorbindungstest wurden die Membranen in Inkubationspuffer (50 mM Tris-HCl, pH 7,4 mit 120 mM NaCl, 5 mM KCl, 2 mM CaCl₂, 2 mM MgCl₂, 10µM Quinolinol, 0,1 % Ascorbinsäure und 0,1 % BSA) in einer Konzentration von ca. 10⁶ Zellen/250 µl Testansatz suspendiert und bei 30°C mit 0,1 nM ¹²⁵Jodsulpirid in Anwesenheit und Abwesenheit von Testsubstanz inkubiert. Die unspezifische Bindung wurde mit 10⁻⁶M Spiperon bestimmt.

Nach 60 min wurde der freie und der gebundene Radioligand durch Filtration über GF/B Glasfaserfilter (Whatman, England) an einem Skatron-Zellsammler (Skatron, Lier, Norwegen) getrennt und die Filter mit eiskaltem Tris-HCl-Puffer, pH 7,4 gewaschen. Die auf den Filtern gesammelte Radioaktivität wurde mit einem Packard 2200 CA Flüssigkeitszintillationszähler quantifiziert.

Die Bestimmung der Kᵢ-Werte erfolgte über nichtlineare Regressionsanalyse mit dem Programm LIGAND.

### 2) D₂-Bindungstest

### Membranpräparationen

a) Nucleus caudatus (Rind)
   Nucleus caudatus wurde aus Rinderhirn entfernt und in eiskalter 0,32 M Saccharose-Lösung gewaschen. Nach Gewichtsbestimmung wurde das Material zerkleinert und in 5 - 10 Volumen Saccharose-Lösung mit einem Potter-Elvehjem Homogenisator (500 U/min) homogenisiert. Das Homogenat wurde bei 3000 x g 15 Minuten (4°C) zentrifugiert und der resultierende Überstand einer weiteren 15minütigen Zentrifugation bei 40000 x g unterworfen. Danach wurde der Rückstand zweimal mit 50 mM Tris-HCl, pH 7,4 durch Resuspension und Zentrifugation gewaschen. Die Membranen wurden bis zum Gebrauch in flüssigem N₂ gelagert.
b) Striatum (Ratte)
   Striati von Sprague-Dawley Ratten wurden in eiskalter 0,32 M Saccharose-Lösung gewaschen. Nach Gewichtsbestimmung wurden die Hirnteile in 5 - 10 Volumen Saccharose-Lösung mit einem Potter-Elvehjem Homogenisator (500 U/min) homogenisiert. Das Homogenat wurde bei 40000 x g 10 Minuten (4°C) zentrifugiert, danach wurde der Rückstand mit 50 mM Tris-HCl, 0,1 mM EDTA und 0,01 % Ascorbinsäure (pH 7,4) mehrmals durch Resuspension und Zentrifugation gewaschen. Der gewaschene Rückstand wurde mit dem obengenannten Puffer resuspendiert und 20 Minuten bei 37°C inkubiert (zwecks Abbau des endogenen Dopamins). Anschließend wurden die Membranen zweimal mit Puffer gewaschen und in Portionen in flüssigem Stickstoff eingefroren. Die Membranpräparation war raximal 1 Woche stabil.

### Bindungstest

a) ³H-Spiperon (D_{2low})
   Nucleus caudatus-Membranen wurden in Inkubationspuffer (mM: Tris-HCl 50, NaCl 120, KCl 5, MgCl₂ 1, CaCl₂ 2, pH 7,4) aufgenommen. Verschiedene Ansätze von je 1 ml wurden hergestellt:
   - Totale Bindung: 400 µg Membranen + 0,2 nmol/l ³H-Spiperon (Du Pont de Nemours, NET-565).
   - Unspezifische Bindung: wie Ansätze für totale Bindung + 10 µM (+)-Butaclamol.
   - Prüfsubstanz: wie Ansätze für totale Bindung + steigende Konzentrationen von Prüfsubstanz.

   Nach erfolgter Inkubation bei 25°C für 60 Minuten wurden die Ansätze über GF/B Glasfaserfilter (Whatman, England) an einem Skatron-Zellsammler (Fa. Zinsser, Frankfurt) filtriert und die Filter mit eiskaltem 50 mM Tris-HCl-Puffer, pH 7,4 gewaschen. Die auf den Filtern gesammelte Radioaktivität wurde mit einem Packard 2200 CA Flüssigkeitszintillationszähler quantifiziert.
   Die Bestimmung der Kᵢ-Werte erfolgte über nichtlineare Regressionsanalyse mit dem Programm LIGAND oder durch Umrechnung der IC₅₀-werte mit Hilfe der Formel von Cheng und Prusoff.
b) ³H-ADTN (D_{2high})
   Striatummembranen wurden in Inkubationspuffer (50 mM Tris-HCl, pH 7,4, 1 mM MnCl₂ und 0,1 % Ascorbinsäure) aufgenommen.
   Verschiedene Ansätze von je 1 ml wurden hergestellt.
   - Totale Bindung: 300 µg Naßgewicht + 1 nM ³H-ADTN (Du Pont de Nemours, Kundensynthese) + 100 nM SCH 23390 (Belegung von D1-Rezeptoren).
   - Unspezifische Bindung: wie Ansätze für totale Bindung + 50 nM Spiperon.
   - Prüfsubstanz: wie Ansätze für totale Bindung + steigende Konzentrationen von Prüfsubstanz.

   Nach erfolgter Inkubation bei 25°C für 60 Minuten wurden die Ansätze über GF/B Glasfaserfilter (Whatman, England) an einem Skatron-Zellsammler (Fa. Zinsser, Frankfurt) filtriert und die Filter mit eiskaltem 50 mM Tris-HCl-Puffer, pH 7,4 gewaschen. Die auf den Filtern gesammelte Radioaktivität wurde mit einem Packard 2200 CA Flüssigkeitszintillationszähler quantifiziert.
   Die Auswertung erfolgte wie unter a).

Die erfindungsgemäßen Verbindungen zeigen in diesen Tests sehr gute Affinitäten und hohe Selektivitäten gegenüber dem D₃-Rezeptor. Die erhaltenen Werte sind für repräsentative Verbindungen in der nachfolgenden Tabelle 9 zusammengestellt.

**Tabelle 9**

| Rezeptorverbindung | | | |
|---|---|---|---|
| Beisp.Nr. | D₃ ¹²⁵ J-Sulpirid Kᵢ[nM] | D₂ ³H-Spiperon Kᵢ[nM] | Selektivität KᵢD₂/KᵢD₃ |
| 10 | 4,5 | 219 | 49 |
| 15 | 8,8 | 517 | 58 |
| 24 | 1,8 | 120 | 67 |
| 41 | 8,1 | 1500 | 185 |
| 42 | 13,4 | 2450 | 182 |
| 37 | 1,7 | 300 | 176 |

Zum Vergleich wurde die Verbindung der Formel 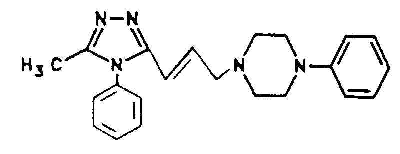 (US 4,577,020; Beispiel 3) dem obigen D₃-Bindungtest unterzogen. Es wurde ein Kᵢ-Wert von 4100 [nM] gefunden, d.h. die Verbindung besitzt praktisch keine Affinität zum D₃-Rezeptor.

## Patentansprüche

1. Triazolverbindungen der Formel I: 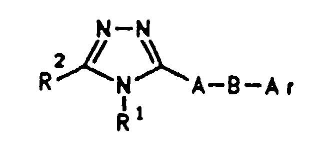 worin
A für eine geradkettige oder verzweigte C₁-C₁₈-Alkylengruppe steht, die gegebenenfalls wenigstens eine Gruppe umfassen kann, die ausgewählt ist unter O, S, NR³, CONR³, NR³CO, COO, OCO, C₃-C₆-Cycloalkylen oder einer Doppel- oder Dreifachbindung,
B für einen Rest der Formel steht: 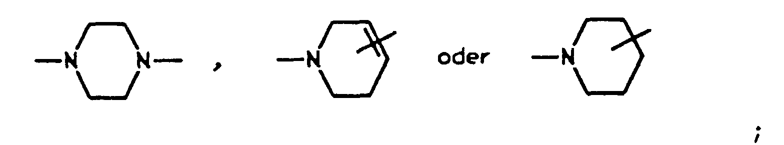
R¹ für H, CO₂R³, NR³R⁴, OR⁴, C₃-C₆-Cycloalkyl oder C₁-C₈-Alkyl, das gegebenenfalls durch OH, OC₁-C₈-Alkyl oder Halogen substituiert ist, steht;
R² die für R¹ angegebenen Bedeutungen besitzt oder für CF₃, SR³, Halogen oder CN steht;
R³ für H oder C₁-C₈-Alkyl, das gegebenenfalls durch OH, OC₁-C₈-Alkyl, Phenyl oder Halogen substituiert ist, steht;
R⁴ die für R³ angegebenen Bedeutungen besitzt oder für COR³ oder CO₂R³ steht;
Ar für Phenyl, Pyridyl, Pyrimidyl oder Triazinyl steht, wobei Ar gegebenenfalls ein bis vier Substituenten aufweisen kann, die unabhängig voneinander ausgewählt sind unter OR⁴, C₁-C₈-Alkyl, C₂-C₆-Alkenyl, C₂-C₆-Alkinyl, Halogen, CN, CO₂R³, NO₂, SO₂R³, SO₃R³, NR³R⁴, SO₂NR³R⁴, SR³, CF₃, CHF₂, einem 5- oder 6-gliedrigen carbocyclischen, aromatischen oder nicht-aromatischen Ring und einem 5- oder 6-gliedrigen heterocyclischen aromatischen oder nicht-aromatischen Ring mit 1 bis 3 Heteroatomen, die ausgewählt sind unter 0, S und N, wobei der carbocyclische oder heterocyclische Ring gegebenenfalls substituiert sein kann durch C₁-C₈-Alkyl, Halogen, OC₁-C₈-Alkyl, OH, NO₂ oder CF₃ und wobei Ar gegebenenfalls auch mit einem carbocyclischen oder heterocyclischen Ring der oben definierten Art kondensiert sein kann,
ausgenommen die Verbindung der Formel: 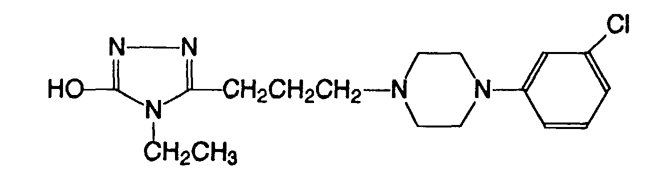
sowie deren Salze mit physiologisch verträglichen Säuren.

2. Verbindungen nach Anspruch 1 der Formel I, worin
R¹ für H, CO₂R³, NR³R⁴, OR⁴ oder C₁-C₈-Alkyl, das gegebenenfalls durch OH, OC₁-C₈-Alkyl oder Halogen substituiert ist, steht;
R³ für H oder C₁-C₈-Alkyl, das gegebenenfalls durch OH, OC₁-C₈-Alkyl oder Halogen substituiert ist, steht;
Ar für Phenyl, Pyridyl, Pyrimidyl oder Triazinyl steht, wobei Ar gegebenenfalls einen oder zwei Substituenten aufweisen kann, die unabhängig voneinander ausgewählt sind unter OR⁴, C₁-C₈-Alkyl, Halogen, CN, CO₂R³, NO₂, SO₂R³, SO₃R³, NR³R⁴, SO₂NR³R⁴, SR³, CF₃, CHF₂, einem 5- oder 6-gliedrigen carbocyclischen, aromatischen oder nicht-aromatischen Ring und einem 5- oder 6-gliedrigen heterocyclischen aromatischen oder nicht-aromatischen Ring mit 1 bis 3 Heteroatomen, die ausgewählt sind unter 0, S und N, wobei der carbocyclische oder heterocyclische Ring gegebenenfalls substituiert sein kann durch C₁-C₈-Alkyl, Halogen, OC₁-C₈-Alkyl, OH, NO₂ oder CF₃ und wobei Ar gegebenenfalls auch mit einem carbocyclischen oder heterocyclischen Ring der oben definierten Art kondensiert sein kann, und
A, B, R² und R⁴ die in Anspruch 1 angegebenen Bedeutungen besitzen.

3. Verbindungen nach Anspruch 1 oder 2 der Formel I, worin A für C₁-C₁₀-Alkylen steht, das gegebenenfalls wenigstens eine Gruppe umfaßt, die ausgewählt ist unter O, S, NR³, Cyclohexylen und einer Doppel- oder Dreifachbindung.

4. Verbindungen nach einem der Ansprüche 1 bis 3 der Formel I, worin
R¹ für H, OR⁴, wobei R⁴ für H oder C₁-C₈-Alkyl steht, C₃-C₆-Cycloalkyl oder C₁-C₈-Alkyl, das gegebenenfalls durch OH, OC₁-C₈-Alkyl oder Halogen substituiert ist, steht;
R² für H, C₁-C₈-Alkyl, das gegebenenfalls durch OH, OC₁-C₈-Alkyl oder Halogen substituiert ist, NR³R⁴, wobei R³ und R⁴ unabhängig voneinander für H, Phenyl-C₁-C₈-alkyl oder C₁-C₈-Alkyl stehen, OR⁴, wobei R⁴ für H oder C₁-C₈-Alkyl steht, oder CF₃ steht; und
Ar für Phenyl, Pyridyl oder Pyrimidyl steht, das gegebenenfalls einen, zwei, drei oder vier Substituenten aufweist, die ausgewählt sind unter H, C₁-C₈-Alkyl, das gegebenenfalls durch OH, OC₁-C₈-Alkyl oder Halogen substituiert ist, OR⁴, wobei R⁴ für H, C₁-C₈-Alkyl, das gegebenenfalls durch OH, OC₁-C₈-Alkyl oder Halogen substituiert ist, CHF₂, CF₃, CN, Halogen, C₂-C₆-Alkenyl, C₂-C₆-Alkinyl, C₅-C₆-Cycloalkyl, Phenyl, Naphthyl und einem 5- oder 6-gliedrigen heterocyclischen aromatischen Rest mit 1 bis 3 Heteroatomen, ausgewählt unter O, N und S.

5. Verbindungen nach einem der Ansprüche 1 bis 3 der Formel I, worin
R¹ für H oder C₁-C₈-Alkyl, das gegebenenfalls substituiert ist durch OH, OC₁-C₈-Alkyl oder Halogen, steht;
R² für H, C₁-C₈-Alkyl, das gegebenenfalls durch OH, OC₁-C₈-Alkyl oder Halogen substituiert ist, NR³R⁴, wobei R³ und R⁴ unabhängig voneinander für H oder C₁-C₈-Alkyl stehen, OR⁴, wobei R⁴ für H oder C₁-C₈-Alkyl steht, oder CF₃ steht;
A für C₁-C₁₀-Alkylen steht, das gegebenenfalls ein Sauerstoff- oder Schwefelatom oder die Gruppe NR³ umfaßt, wobei R³ wie oben definiert ist;
Ar für Phenyl steht, das ein bis vier Substituenten aufweist, die unabhängig voneinander ausgewählt sind unter H, CN, SR³, Halogen, C₁-C₈-Alkyl, das gegebenenfalls durch OH, OC₁-C₈-Alkyl oder Halogen substituiert ist, Phenyl, Naphthyl, OR⁴, NO₂, NR³R⁴, CHF₂ und CF₃, wobei R³ und R⁴ die angegebenen Bedeutungen besitzen.

6. Verbindungen nach Anspruch 5 der Formel I, worin
A für SC₃-C₁₀-Alkylen, OC₃-C₁₀-Alkylen oder NR³-C₃-C₁₀-Alkylen steht, wobei R³ für H oder C₁-C₈-Alkyl steht;
R¹ für H oder C₁-C₈-Alkyl steht;
R² die in Anspruch 5 angegebenen Bedeutungen besitzt;
B für 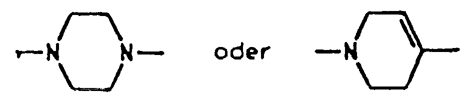 steht:
Ar für Phenyl steht, das ein bis vier Substituenten aufweist, die unabhängig voneinander für H, C₁-C₈-Alkyl, OC₁-C₈-Alkyl, CHF₂, CF₃ oder CN stehen.

7. Verbindungen nach Anspruch 6, wobei Ar einen oder zwei Substituenten aufweist, die sich in 3-Stellung bzw. 5-Stellung befinden, wobei der eine Substituent CF₃, CHF₂ oder C₁-C₈-Alkyl ist und der andere Substituent H oder C₁-C₈-Alkyl ist.

8. Verbindungen nach Anspruch 1 der Formel I, worin
Ar für Pyrimidinyl steht, das ein bis drei Substituenten aufweist, die unabhängig voneinander ausgewählt sind unter H, C₁-C₈-Alkyl, Phenyl, Naphthyl, C₅-C₆-Cycloalkyl, OH, OC₁-C₈-Alkyl, Halogen, CN, CF₃, CHF₂ und einem 5- oder 6-gliedrigen, heterocyclischen, aromatischen Rest mit 1 bis 3 Heteroatomen, ausgewählt unter O, N und S;
R¹ für H oder C₁-C₈-Alkyl, das gegebenenfalls durch OH, OC₁-C₈-Alkyl oder Halogen substituiert ist, steht;
R² für H, NR³R⁴ oder OR⁴ steht, wobei R³ und R⁴ unabhängig voneinander für H, C₁-C₈-Alkyl oder Phenyl-C₁-C₈-alkyl stehen;
A für C₁-C₁₀-Alkylen steht, das gegebenenfalls wenigstens eine Gruppe umfaßt, die ausgewählt ist unter O, S, NR³, wobei R³ für H oder C₁-C₈-Alkyl steht, und einer Doppel- oder Dreifachbindung; und
B wie in Anspruch 1 definiert ist.

9. Verbindungen nach Anspruch 1 der Formel I, worin
Ar für Pyridinyl steht, das ein bis vier Substituenten aufweist, die unabhängig voneinander ausgewählt sind unter H, C₁-C₈-Alkyl, Phenyl, Naphthyl, OH, OC₁-C₈-Alkyl, Halogen, CF₃, CN, C₂-C₆-Alkenyl, C₂-C₆-Alkinyl und einem 5- oder 6-gliedrigen, heterocyclischen, aromatischen Rest mit 1 bis 3 Heteroatomen, ausgewählt unter O, N und S;
R¹ für H, C₁-C₈-Alkyl, C₃-C₆-Cycloalkyl oder OR⁴ steht, wobei R⁴ für H oder C₁-C₈-Alkyl, das gegebenenfalls durch OH, OC₁-C₈-Alkyl oder Halogen substituiert ist, steht; und
R², A und B wie in Anspruch 8 definiert sind.

10. Verfahren zur Herstellung der Verbindungen nach einem der vorhergehenden Ansprüche, dadurch gekennzeichnet, daß man
i) eine Verbindung der allgemeinen Formel II: 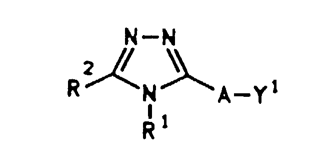 worin
Y¹ für eine übliche Abgangsgruppe steht, mit einer Verbindung der allgemeinen Formel III
H - B - Ar
umsetzt;
ii) zur Herstellung einer Verbindung der Formel I,
worin A ein Sauerstoff- oder Schwefelatom oder NR³ umfaßt:
a) eine Verbindung der allgemeinen Formel IV: 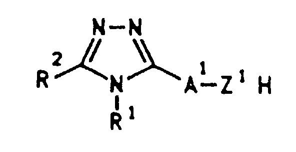 worin Z¹ für O, S oder NR³ steht und A¹ für C_{O}-C₁₈-Alkylen steht, mit einer Verbindung der allgemeinen Formel VI
Y¹ - A² - B - Ar
worin Y¹, die oben angegebenen Bedeutungen besitzt und A² für C₁-C₁₈-Alkylen steht, wobei A¹ und A² zusammen 1 bis 18 Kohlenstoffatome aufweisen, umsetzt;
iii) zur Herstellung einer Verbindung der Formel I, worin A die Gruppe COO oder CONR³ umfaßt:
a) eine Verbindung der allgemeinen Formel VII: 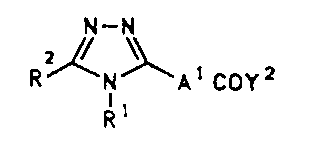 worin Y² für OH, OC₁-C₄-Alkyl, Cl oder zusammen mit CO für eine aktivierte Carboxylgruppe steht, und A¹ die oben angegebenen Bedeutungen besitzt,
mit einer Verbindung der Formel VIII:
Z¹ - A² - B - Ar
worin A² die oben angegebenen Bedeutungen besitzt, und Z¹ für OH oder NHR³ steht,
umsetzt,
iv) zur Herstellung einer Verbindung der Formel I, worin A die Gruppe OCO oder NR³CO umfaßt:
a) eine Verbindung der Formel IV 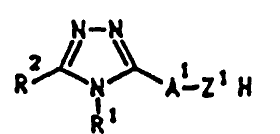 worin Z¹ für o oder NR³ steht, mit einer Verbindung der Formel X:
Y²CO - A² - B - Ar
worin A² und Y² die oben angegebenen Bedeutungen besitzen, umsetzt, wobei in den obigen Formeln R¹, R², A, B und Ar die in Anspruch 1 angegebenen Bedeutungen besitzen.

11. Pharmazeutisches Mittel, enthaltend mindestens eine Verbindung der Formel I nach einem der Ansprüche 1 bis 9, wobei jedoch die in Anspruch 1 genannte Ausnahme nicht gilt, gegebenenfalls zusammen mit physiologisch akzeptablen Trägern und/oder Hilfsstoffen.

12. Verwendung wenigstens einer Verbindung der Formel I nach einem der Ansprüche 1 bis 9, wobei jedoch die in Anspruch 1 genannte Ausnahme nicht gilt, zur Herstellung eines pharmazeutischen Mittels zur Behandlung von Erkrankungen, die auf Dopamin-D₃-Rezeptorliganden ansprechen.

## Claims

1. A triazole compound of the formula I: 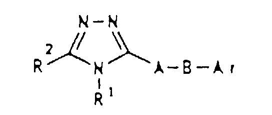 where
A is a straight-chain or branched C₁-C₁₈-alkylene group which may comprise at least one group selected from O, S, NR³, CONR³, NR³CO, COO, OCO, C₃-C₆-cycloalkylene or a double or triple bond,
B is a radical of the formula: 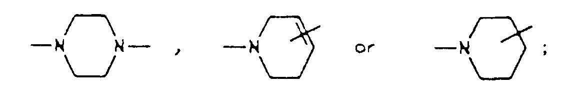
R¹ is H, CO₂R³, NR³R⁴, OR⁴, C₃-C₆-cycloalkyl or C₁-C₈-alkyl which is unsubstituted or substituted by OH, OC₁-C₈-alkyl or halogen;
R² has the meanings indicated for R¹ or is CF₃, SR³, halogen or CN;
R³ is H or C₁-C₈-alkyl which is unsubstituted or substituted by OH, OC₁-C₈-alkyl, phenyl or halogen;
R⁴ has the meanings indicated for R³ or is COR³ or CO₂R³;
Ar is phenyl, pyridyl, pyrimidyl or triazinyl, where Ar may have from one to four substituents which are selected, independently of one another, from OR⁴, C₁-C₈-alkyl, C₂-C₆-alkenyl, C₂-C₆-alkynyl, halogen, CN, CO₂R³, NO₂, SO₂R³, SO₃R³, NR³R⁴, SO₂NR³R⁴, SR³, CF₃, CHF₂, a 5- or 6-membered carbocyclic aromatic or nonaromatic ring and a 5- or 6-membered heterocyclic aromatic or nonaromatic ring having 1 to 3 hetero atoms selected from O, S and N, where the carbocyclic or heterocyclic ring may be unsubstituted or substituted by C₁-C₈-alkyl, halogen, OC₁-C₈-alkyl, OH, NO₂ or CF₃ and where Ar may also be fused to a carbocyclic or heterocyclic ring of the type defined above,
with the exception of the compound of the formula: 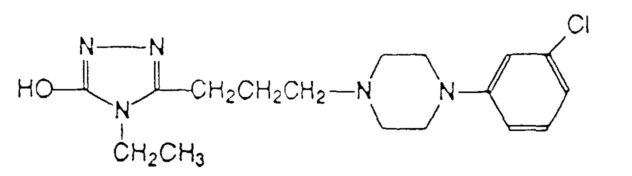
and the salts thereof with physiologically tolerated acids.

2. A compound as claimed in claim 1 of the formula I where
R¹ is H, CO₂R³, NR³R⁴, OR⁴ or C₁-C₈-alkyl which is unsubstituted or substituted by OH, OC₁-C₈-alkyl or halogen;
R³ is H or C₁-C₈-alkyl which is unsubstituted or substituted by OH, OC₁-C₈-alkyl or halogen;
Ar is phenyl, pyridyl, pyrimidyl or triazinyl, where Ar may have one or two substituents which are selected, independently of one another, from OR⁴, C₁-C₈-alkyl, halogen, CN, CO₂R³, NO₂, SO₂R³, SO₃R³, NR³R⁴, SO₂NR³R⁴, SR³, CF₃, CHF₂, a 5- or 6-membered carbocyclic aromatic or nonaromatic ring and a 5- or 6-membered heterocyclic aromatic or nonaromatic ring with 1 to 3 hetero atoms selected from O, S and N, where the carbocyclic or heterocyclic ring may be unsubstituted or substituted by C₁-C₈-alkyl, halogen, OC₁-C₈-alkyl, OH, NO₂ or CF₃ and where Ar may also be fused to a carbocylic or heterocyclic ring of the type defined above, and
A, B, R² and R⁴ have the meanings stated in claim 1.

3. A compound as claimed in claim 1 or 2 of the formula I where A is C₁-C₁₀-alkylene which may comprise at least one group selected from O, S, NR³, cyclohexylene and a double or triple bond.

4. A compound as claimed in any of claims 1 to 3 of the formula I where
R¹ is H, OR⁴ where R⁴ is H or C₁-C₈-alkyl, or C₃-C₆-cycloalkyl or C₁-C₈-alkyl which is unsubstituted or substituted by OH, OC₁-C₈-alkyl or halogen;
R² is H, C₁-C₈-alkyl which is unsubstituted or substituted by OH, OC₁-C₈-alkyl or halogen, or NR³R⁴ where R³ and R⁴ are, independently of one another, H, phenyl-C₁-C₈-alkyl or Cₗ-C₈-alkyl, or OR⁴ where R⁴ is H or C₁-C₈-alkyl, or CF₃; and
Ar is phenyl, pyridyl or pyrimidyi which may have one, two, three or four substituents which are selected from H, C₁-C₈-alkyl which is unsubstituted or substituted by OH, OC₁-C₈-alkyl or halogen, or OR⁴ where R⁴ is H, C₁-C₈-alkyl which is unsubstituted or substituted by OH, OC₁-C₈-alkyl or halogen, or CHF₂, CF₃; CN, halogen, C₂-C₆-alkenyl, C₂-C₆-alkynyl, C₅-C₆-cycloalkyl, phenyl, naphthyl and a 5- or 6-membered heterocyclic aromatic radical with 1 to 3 hetero atoms selected from O, N and S.

5. A compound as claimed in any of claims 1 to 3 of the formula I where
R¹ is H or C₁-C₈-alkyl which is unsubscituted or substituted by OH, OC₁-C₈-alkyl or halogen;
R² is H, C₁-C₈-alkyl which is unsubstituted or substituted by OH, OC₁-C₈-alkyl or halogen, or NR³R⁴ where R³ and R⁴ are, independently of one another, H or C₁-C₈-alkyl, or OR⁴ where R⁴ is H or C₁-C₈-alkyl, or CF₃;
A is C₁-C₁₀-alkylene which may comprise an oxygen or sulfur atom or the group NR³ where R³ is as defined above;
Ar is phenyl which may have one to four substituents which are selected, independently of one another, from H, CN, SR³, halogen, C₁-C₈-alkyl which is unsubstituted or substituted by OH, OC₁-C₈-alkyl or halogen, or phenyl, naphthyl, OR⁴, NO₂, NR³R⁴, CHF₂ and CF₃, where R³ and R⁴ have the stated meanings.

6. A compound as claimed in claim 5 of the formula I where
A is SC₃-C₁₀-alkylene, OC₃-C₁₀-alkylene or NR³-C₃-C₁₀-alkylene, where R³ is H or C₁-C₈-alkyl;
R¹ is H or C₁-C₈-alkyl;
R² has the meanings stated in claim 5;
B is: 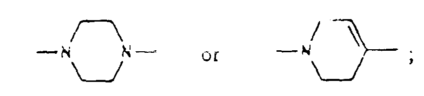
Ar is phenyl which has one to four substituents which are, independently of one another, H, C₁-C₈-alkyl, OC₁-C₈-alkyl, CHF₂, CF₃ or CN.

7. A compound as claimed in claim 6, where Ar has one or two substituents which are located in position 3 and position 5, with one substituent being CF₃, CHF₂ or C₁-C₈-alkyl and the other substituent being H or C₁-C₈-alkyl.

8. A compound as claimed in claim 1 of the formula I where
Ar is pyrimidinyl which has one to three substituents which are selected, independently of one another, from H, C₁-C₈-alkyl, phenyl, naphthyl, C₅-C₆-cycloalkyl, OH, OC₁-C₈-alkyl, halogen, CN, CF₃, CHF₂ and a 5- or 6-membered heterocyclic aromatic radical with 1 to 3 hetero atoms selected from O, N and S;
R¹ is H or C₁-C₈-alkyl which is unsubstituted or substituted by OH, OC₁-C₈-alkyl or halogen,
R² is H, NR³R⁴ or OR⁴ where R³ and R⁴ are, independently of one another, H, C₁-C₈-alkyl or phenyl-C₁-C₈-alkyl;
A is C₁-C₁₀-alkylene which may comprise at least one group selected from O, S, NR³ where R³ is H or C₁-C₈-alkyl, and a double or triple bond; and
B is as defined in claim 1.

9. A compound as claimed in claim 1 of the formula I where
Ar is pyridinyl which has one to four substituents which are selected, independently of one another, from H, C₁-C₈-alkyl, phenyl, naphthyl, OH, OC₁-C₈-alkyl, halogen, CF₃, CN, C₂-C₆-alkenyl, C₂-C₆-alkynyl and a 5- or 6-membered heterocyclic aromatic radical with 1 to 3 hetero atoms selected from O, N and S;
R¹ is H, C₁-C₈-alkyl, C₃-C₆-cycloalkyl or OR⁴ where R⁴ is H or C₁-C₈-alkyl which is unsubstituted or substituted by OH, OC₁-C₈-alkyl or halogen; and
R², A and B are as defined in claim 8.

10. A process for preparing compounds as claimed in any of the preceding claims, which comprises
i) reacting a compound of the general formula II: 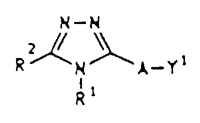 where Y¹ is a conventional leaving group, with a compound of the general formula III
H - B - Ar;
ii) to prepare a compound of the formula I where A is an oxygen or sulfur atom or NR³:
a) reacting a compound of the general formula IV: 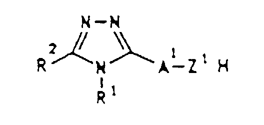 where Z¹ is O, S or NR³ and A¹ is C₀-C₁₈-alkylene, with a compound of the general formula VI
Y¹ - A² - B - Ar
where Y¹ has the abovementioned meanings, and A² is C₁-C₁₈-alkylene, where A¹ and A² together have 1 to 18 carbon atoms;
iii) to prepare a compound of the formula I where A comprises the group COO or CONR³:
a) reacting a compound of the general formula VII: 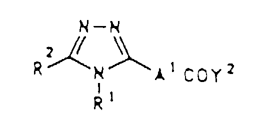 where Y² is OH, OC₁-C₄-alkyl, Cl or, together with CO, is an activated carboxyl group, and A¹ has the abovementioned meanings, with a compound of the formula VIII:
Z¹ - A² - B - Ar
where A² has the abovementioned meanings, and Z¹ is OH or NHR³,
iv) to prepare a compound of the formula I where A comprises the group OCO or NR³CO:
a) reacting a compound of the formula IV 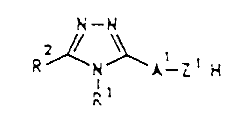 where Z¹ is O or NR³, with a compound of the formula X:
Y²CO - A² - B - Ar
where A² and Y² have the abovementioned meanings and where R¹, R², A, B and Ar in the above formulae have the meanings stated in claim 1.

11. A pharmaceutical composition comprising at least one compound of formula I as claimed in any of claims 1 to 9, whereby the disclaimer in claim 1 is not valid, with or without physiologically acceptable vehicles and/or ancillary substances.

12. The use of at least one compound of formula I as claimed in any of claims 1 to 9, whereby the disclaimer in claim 1 is not valid, for the preparation of a pharmaceutical composition for treating disorders responding to dopamine D₃ receptor ligands.

## Revendications

1. Dérivés triazoliques de formule I 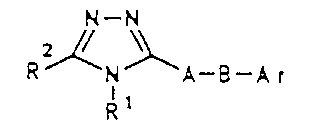 dans laquelle
A représente un groupe alkylène à chaîne droite ou ramifiée en C1-C18 qui peut contenir le cas échéant au moins un constituant choisi parmi O, S, NR³, CONR³, NR³CO, COO, OCO, un groupe cycloalkylène en C3-C6 ou une double ou triple liaison,
B représente un groupe de formule 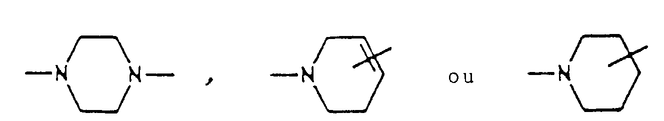
R¹ représente H, CO₂R³, NR³R⁴, OR⁴, un groupe cycloalkyle en C3-C6 ou alkyle en C1-C8, qui peut éventuellement porter un substituant OH, Oalkyle en C1-C8 ou halogéno ;
R² a les significations indiquées pour R¹ ou représente CF₃, SR³, un halogène ou CN ;
R³ représente H ou un groupe alkyle en C1-C8 qui peut le cas échéant porter un substituant OH, Oalkyle en C1-C8, phényie ou halogéno ;
R⁴ a les significations indiquées pour R³ ou représente COR³ ou CO₂R³ ;
Ar représente un groupe phényle, pyridyle, pyrimidyle ou triazinyle, le groupe Ar pouvant le cas échéant porter un à quatre substituants choisis, indépendamment les uns des autres, parmi OR⁴, les groupes alkyle en C1-C8, alcényle en C2-C6, alcynyle en C2-C6, les halogènes, CN, CO₂R³, NO₂, SO₂R³, SO₃R³, NR³R⁴, SO₂NR³R⁴, SR³, CF₃, CHF_{3,} un noyau carbocyclique aromatique ou non aromatique à cinq ou six chaînons, et un noyau hétérocyclique aromatique ou non aromatique à cinq ou six chaînons contenant un à trois éléments choisis parmi O, S et N, le noyau carbocyclique ou hétérocyclique pouvant le cas échéant porter un substituant alkyle en C1-C8, halogéno, Oalkyle en C1-C8, OH, NO₂ ou CF₃, le groupe Ar pouvant également être condensé le cas échéant avec un noyau carbocyclique ou hétérocyclique du type défini ci-dessus,
à l'exception du composé de formule 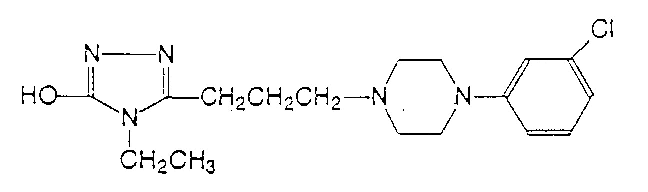 et leurs sels d'acides acceptables pour l'usage pharmaceutique.

2. Composés selon la revendication 1, répondant à la formule I dans laquelle
R¹ représente H, CO₂R³, NR³R⁴, OR⁴ ou un groupe alkyle en C1-C8 qui peut le cas échéant porter des substituants OH, Oalkyle en C1-C8 où halogéno ;
R³ représente H ou un groupe alkyle en C1-C8 qui peut le cas échéant porter des substituants OH, Oalkyle en C1-C8 ou halogéno ;
Ar représente un groupe phényle, pyridyle, pyrimidyle ou triazinyle, le groupe Ar pouvant le cas échéant porter un ou deux substituants choisis, indépendamment les uns des autres, parmi OR⁴, les groupes alkyle en C1-C8, les halogènes, CN, CO₂R³, NO₂, SO₂R³, SO₃R³, NR³R⁴, SO₂NR³R⁴, SR³, CF₃, CHF₂, un noyau carbocyclique aromatique ou non aromatique à cinq ou six chaînons et un noyau hétérocyclique aromatique ou non aromatique à cinq ou six chaînons contenant un à trois hétéroatomes choisis parmi O, S et N, le noyau carbocyclique ou hétérocyclique pouvant le cas échéant porter des substituants alkyle en C1-C8, halogéno, Oalkyle en C1-C8, OH, NO₂ ou CF₃, le groupe Ar pouvant le cas échéant être également condensé avec un noyau carbocyclique ou hétérocyclique du type défini ci-dessus, et
A, B, R² et R⁴ ont les significations indiquées dans la revendication 1.

3. Composés selon la revendication 1 ou 2, répondant à la formule I dans laquelle A représente un groupe alkylène en C1-C10 comprenant le cas échéant au moins un constituant choisi parmi O, S, NR³, un groupe cyclohexylène ou une double ou triple liaison.

4. Composés selon une des revendications 1 à 3, répondant à la formule I dans laquelle
R¹ représente H, OR⁴ dans lequel R⁴ représente H ou un groupe alkyle en C1-C8, cycloalkyle en C3-C6 ou alkyle en C1-C8 portant le cas échéant des substituants OH, Oalkyle en C1-C8 ou halogéno ;
R² représente H, un groupe alkyle en C1-C8 qui peut le cas échéant porter des substituants OH, Oalkyle en C1-C8 ou halogéno, NR³R⁴ dans lequel R³ et R⁴ représentent chacun, indépendamment l'un de l'autre, H, un groupe phényl-alkyle en C1-C8 ou alkyle en C1-C8, OR⁴ dans lequel R⁴ représente H ou un groupe alkyle en C1-C8 ou CF₃ ; et
Ar représente un groupe phényle, pyridyle ou pyrimidyle qui porte le cas échéant un, deux, trois ou quatre substituants choisis parmi H, les groupes alkyle en C1-C8 portant éventuellement des substituants OH, Oalkyle en C1-C8 ou halogéno, les groupes OR⁴ dans lesquels R⁴ représente H, un groupe alkyle en C1-C8 portant éventuellement des substituants OH, Oalkyle en C1-C8 ou halogéno, les groupes CHF₂, CF₃, CN, les halogènes, les groupes alcényle en C2-C6, alcynyle en C2-C6, cycloalkyle en C5-C6, phényle, naphtyle, et un radical hétérocyclique aromatique à cinq ou six chaînons contenant un à trois hétéroatomes choisis parmi O, N et S.

5. Composés selon une des revendications 1 à 3, répondant à la formule I dans laquelle
R¹ représente H ou un groupe alkyle en C1-C8 portant éventuellement des substituants OH, Oalkyle en C1-C8 ou halogéno ;
R² représente H, un groupe alkyle en C1-C8 portant éventuellement des substituants OH, Oalkyle en C1-C8 ou halogéno, un groupe NR³R⁴ dans lequel R³ et R⁴ représentent chacun, indépendamment l'un de l'autre, H ou un groupe alkyle en C1-C8, un groupe OR⁴ dans lequel R⁴ représente H ou un groupe alkyle en C1-C8, ou le groupe CF₃ ;
A représente un groupe alkylène en C1-C10 contenant le cas échéant un atome d'oxygène ou de soufre ou le groupe NR³ dans lequel R³ est tel que défini ci-dessus ;
Ar représente un groupe phényle portant un à quatre substituants choisis, indépendamment les uns des autres, parmi H, CN, SR³, les halogènes, les groupes alkyle en C1-C8 portant éventuellement des substituants OH, Oalkyle en C1-C8 ou halogéno, les groupes phényle, naphtyle, OR⁴, NO₂, NR³R⁴, CHF₂ et CF₃, dans lesquels R³ et R⁴ ont les significations indiquées ci-dessus.

6. Composés selon la revendication 5, répondant à la formule I dans laquelle
A représente un groupe Salkylène en C3-C10, Oalkylène en C3-C10 ou NR³-alkylène en C3-C10, dans lequel R³ représente H ou un groupe alkyle en C1-C8 ;
R¹ représente H ou un groupe alkyle en C1-C8 ;
R² a les significations indiquées dans la revendication 5 ;
B représente 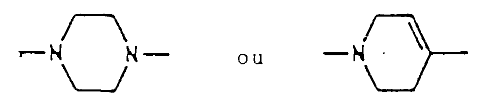
Ar représente un groupe phényle portant un à quatre substituants choisis, indépendamment les uns des autres, parmi H, les groupes alkyle en C1-C8, Oalkyle en C1-C8, CHF₂, CF₃ ou CN.

7. Composés selon la revendication 6, pour lesquels Ar porte un ou deux substituants se trouvant dans les positions respectives 3 et 5, l'un des substituants consistant en CF₃, CHF₂ ou un groupe alkyle en C1-C8 et l'autre substituant en H ou un groupe alkyle en C1-C8.

8. Composés selon la revendication 1, répondant à la formule I dans laquelle
Ar représente un groupe pyrimidinyle portant un à trois substituants choisis, indépendamment les uns des autres, parmi H, les groupes alkyle en C1-C8, phényle, naphtyle, cycloalkyle en C5-C6, OH, Oalkyle en C1-C8, les halogènes, CN, CF₃, CHF₂ et un radical hétérocyclique aromatique à cinq ou six chaînons contenant un à trois hétéroatomes choisis parmi O, N et S ;
R¹ représente H ou un groupe alkyle en C1-C8 éventuellement substitué par OH, Oalkyle en C1-C8 ou un halogène ;
R² représente H, NR³R⁴ ou OR⁴ dans lesquels R³ et R⁴ représentent chacun, indépendamment l'un de l'autre, H, un groupe alkyle en C1-C8 ou phényl-alkyle en C1-C8 ;
A représente un groupe alkylène en C1-C10 comprenant le cas échéant au moins un constituant choisi parmi O, S, NR³ dans lequel R³ représente H ou un groupe alkyle en C1-C8, et une double ou triple liaison ; et
B a les significations indiquées dans la revendication 1.

9. Composés selon la revendication 1 répondant à la formule I dans laquelle
Ar représente un groupe pyridinyle portant un à quatre substituants choisis, indépendamment les uns des autres, parmi H, les groupes alkyle en C1-C8, phényle, naphtyle, OH, Oalkyle en C1-C8, les halogènes, CF₃, CN, les groupes alcényle en C2-C6, alcynyle en C2-C6 et un radical hétérocyclique aromatique à cinq ou six chaînons contenant un à trois hétéroatomes parmi O, N et S ;
R¹ représente H, un groupe alkyle en C1-C8, cycloalkyle en C3-C6 ou OR⁴ dans lequel R⁴ représente H ou un groupe alkyle en C₁-C₈ lui-même éventuellement substitué par OH, Oalkyle en C1-C8 ou halogéno ; et
R², A et B ont les significations indiquées dans la revendication 8.

10. Procédé de préparation des composés selon l'une des revendications qui précèdent, caractérisé par le fait que
i) on fait réagir un composé de formule générale II 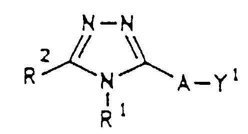 dans laquelle
Y¹ représente un groupe éliminable usuel, avec un composé de formule générale III
H - B - Ar
ii) pour la préparation d'un composé de formule I dans laquelle A représente un atome d'oxygène ou de soufre ou NR³ :
a) on fait réagir un composé de formule générale IV 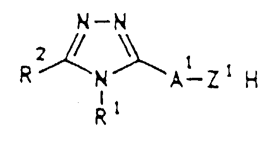 dans laquelle Z¹ représente O, S ou NR³ et A¹ représente un groupe alkylène en C0-C18 avec un composé de formule générale VI
Y¹ - A2 - B - Ar
dans laquelle Y¹ a les significations indiquées ci-dessus et A² représente un groupe aikylène en C1-C18, A¹ et A² pouvant contenir ensemble de 1 à 18 atomes de carbone ;
iii) pour la préparation d'un composé de formule I dans laquelle A représente un groupe COO ou CONR³ :
a) on fait réagir un composé de formule générale VII 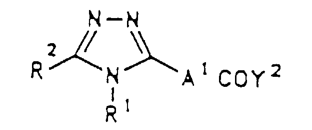 dans laquelle Y² représente OH, Oalkyle en C1-C4, Cl, ou forme avec CO un groupe carboxyle activé, et A¹ a les significations indiquées ci-dessus, avec un composé de formule VIII
Z¹ - A2 - B - Ar
dans laquelle A² a les significations indiquées ci-dessus et Z¹ représente OH ou NHR³;
iv) pour la préparation d'un composé de formule I dans laquelle A représente un groupe OCO ou NR³CO :
a) on fait réagir un composé de formule IV 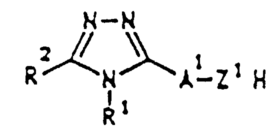 dans laquelle Z¹ représente O ou NR³, avec un composé de formule X
Y²CO - A² - B - Ar
dans laquelle A² et Y² ont les significations indiquées ci-dessus, les symboles R¹, R², A, B et Ar des formules ci-dessus ayant les significations indiquées dans la revendication 1.

11. Produit pharmaceutique contenant au moins un composé de formule I selon une des revendications 1 à 9 mais sans l'exception mentionnée dans la revendication 1, le cas échéant avec des véhicules et/ou produits auxiliaires acceptables pour l'usage pharmaceutique.

12. Utilisation d'au moins un composé de formule I selon une des revendications 1 à 9, mais sans l'exception mentionnée dans la revendication 1, pour la préparation d'un produit pharmaceutique prévu pour le traitement de maladies demandant des ligands des récepteurs D₃ de la dopamine.
